# EUROPEAN PATENT SPECIFICATION

(11) **EP 2 865 345 B1**
(45) Date of publication and mention of the grant of the patent: **28.12.2016**
(21) Application number: 13862066.1
(22) Date of filing: 08.11.2013
(51) Int. Cl.: A61B 17/34, A61B 1/00, A61B 8/12, A61B 18/14

(54) **PUNCTURE TOOL AND ULTRASONIC ENDOSCOPE**
PUNKTIONSINSTRUMENT UND ULTRASCHALLENDOSKOP
OUTIL DE PONCTION ET ENDOSCOPE ULTRASONORE

(30) Priority: 12.12.2012 JP 2012271651
(43) Date of publication of application: 29.04.2015
(73) Proprietor: Olympus Corporation, Tokyo 192-8507 (JP)
(72) Inventor: MATSUI, Shoichi, Tokyo 151-0072 (JP); NISHINA, Kenichi, Tokyo 151-0072 (JP); KAJI, Kunihide, Tokyo 151-0072 (JP)
(74) Representative: Schicker, Silvia
(86) International application number: PCT/JP2013/080222
(87) International publication number: WO 2014/091846

(56) References cited:
- JP-A- 2000 116 657
- JP-A- 2002 531 211
- JP-A- 2004 531 290
- US-A- 6 022 362
- US-A1- 2003 109 870
- US-A1- 2008 051 626
- US-A1- 2008 103 504
- US-A1- 2010 063 392

## Description

### Technical Field

The present invention relates to a puncture tool and an ultrasound endoscope, and more particularly to a puncture tool capable of reducing a lesion part while preventing adhesion of living lesion cells at a time of pulling out, and an ultrasound endoscope.

### Background Art

In the past, as treatment methods and suffering relief methods for a lesion part such as a tumor, there have been radiation therapy, chemotherapy, physical treatment and the like. For complete curing of a lesion part such as a tumor, a surgical operation for excising the lesion part is preferable as physical treatment.

For example, in Japanese Patent Application Laid-Open Publication No. 2000-116657, a dissection biopsy apparatus for excising a lesion part is proposed. In the case of the dissection biopsy apparatus, a lesion part is excised by bending a thin ribbon-type cutting tool provided at a distal end portion of the biopsy apparatus and rotating the bent cutting tool.

The excised lesion part is received into a saclike tissue reception apparatus connected to the cutting tool, and the whole dissection biopsy apparatus is pulled out of a body. Thus, the lesion part is excised and taken out.

However, in the case of taking out a biological tissue which includes lesion cells to the outside of a body using the above proposed dissection biopsy apparatus, living lesion cells may adhere to the inside of the biological tissue of a dissected region during a period until the biological tissue is pulled out of the body.

US 2003/109870 discloses an electrically activated surgical device with a similar ribbon-type cutting tool.

Therefore, an object of the present invention is to provide a puncture tool capable of causing a lesion part to be reduced by being punctured into the lesion part and shaving an inside of the lesion part and capable of preventing living lesion cells from adhering to a dissected region at a time of pulling out from the lesion part, and an ultrasound endoscope which includes the puncture tool.

### Disclosure of Invention

### Means for Solving the Problem

According to an aspect of the present invention, it is possible to provide a puncture tool including: a tubular portion internally including a channel whose distal end side opens on a side surface; a suction device connecting portion arranged on a proximal end side of the tubular portion so as to communicate with the channel and connected to a suction device; a puncture portion to be punctured into a subject, the puncture portion being arranged on a distal end face of the tubular portion; an opening portion of the channel provided on a side surface of the puncture portion; a blade capable of being inserted through the channel and capable of causing a cutting portion to project from the opening portion when being inserted through the channel; and a first power supply connecting portion for electrically connecting the puncture portion and a power supply.

According to an aspect of the present invention, it is possible to provide an ultrasound endoscope including: a puncture tool of the present invention, and an ultrasound observation portion transmitting ultrasound toward the puncture tool and receiving the ultrasound reflected from the puncture tool.

### Brief Description of the Drawings

Fig. 1 is a configuration diagram showing a configuration of an operation system using an ultrasound endoscope according to a first embodiment of the present invention;
Fig. 2 is a plan view of a distal end portion of a puncture tool insertion portion 9A according to the first embodiment of the present invention;
Fig. 3 is a sectional view of the distal end portion of the puncture tool insertion portion 9A taken along line III-III in Fig. 2;
Fig. 4 is a sectional view of the distal end portion of the puncture tool insertion portion 9A taken along line IV-IV in Fig. 2;
Fig. 5 is a perspective view of a distal end portion of a blade 22 according to the first embodiment of the present invention;
Fig. 6 is an external view of a puncture tool operation portion 9B according to the first embodiment of the present invention;
Fig. 7 is a sectional view of the puncture tool operation portion 9B taken along line VII-VII in Fig. 6;
Fig. 8 is a sectional view of the puncture tool operation portion 9B in a state in which a sheath 41 projects most from a treatment instrument opening of a distal end rigid portion 14 according to the first embodiment of the present invention;
Fig. 9 is a sectional view of the puncture tool operation portion 9B in a state in which a sheath 41 and a needle tube 21 project most from the treatment instrument opening of the distal end rigid portion 14 according to the first embodiment of the present invention;
Fig. 10 is a sectional view of the puncture tool operation portion 9B in a state in which the sheath 41 and the needle tube 21 project most from the treatment instrument opening of the distal end rigid portion 14 and the blade 22 projects by one stage from the treatment instrument opening of the distal end rigid portion 14 according to the first embodiment of the present invention;
Fig. 11 is a sectional view of the puncture tool operation portion 9B in a state in which the sheath 41 and the needle tube 21 project most from the treatment instrument opening of the distal end rigid portion 14 and the blade 22 projects most from the treatment instrument opening of the distal end rigid portion 14 according to the first embodiment of the present invention;
Fig. 12 is a sectional view showing a state in which a blade distal end portion 22a comes into contact with a distal end portion 21d1 of an internal space 21 d but the blade distal end portion 22a is not bent according to the first embodiment of the present invention;
Fig. 13 is a sectional view showing a state in which the blade distal end portion 22a comes into contact with the distal end portion 21d1 of the internal space 21d, the blade 22 is further pushed out to a distal end side, and the blade distal end portion 22a is bent according to the first embodiment of the present invention;
Fig. 14 is a schematic explanatory diagram of a human body including a pancreas and a stomach according to the first embodiment of the present invention;
Fig. 15 is a diagram for explaining a flow of a manipulation for reducing a tumor part Pa, which is a tumor part of a pancreas P, according to the first embodiment of the present invention;
Fig. 16 is a diagram for explaining a projecting state of a blade distal end portion 22a according to the first embodiment of the present invention;
Fig. 17 is a diagram showing an example of an ultrasound image displayed on a monitor 7 according to the first embodiment of the present invention;
Fig. 18 is a diagram for explaining a movement of the blade distal end portion 22a according to the first embodiment of the present invention;
Fig. 19 is a diagram for explaining injection of saline or ethanol according to the first embodiment of the present invention;
Fig. 20 is a diagram showing an example of an ultrasound image displayed on the monitor 7 when the saline or the ethanol is injected according to the first embodiment of the present invention;
Fig. 21 is a diagram for explaining collection of resected tumor tissue pieces according to the first embodiment of the present invention;
Fig. 22 is a diagram showing an example of an ultrasound image displayed on the monitor 7 when the resected tumor tissue pieces are collected according to the first embodiment of the present invention;
Fig. 23 is a plan view of a needle tube distal end portion 21b1 of a puncture tool insertion portion 9A1 according to a modification 1 of the first embodiment of the present invention;
Fig. 24 is a plan view of a needle tube distal end portion 21b2 of a puncture tool insertion portion 9A2 according to a modification 2 of the first embodiment of the present invention;
Fig. 25 is a sectional view taken along line XXV-XXV in Fig. 24;
Fig. 26 is a perspective view of the blade distal end portion 22a according to a modification 3 of the first embodiment of the present invention;
Fig. 27 is a perspective view of the blade distal end portion 22a according to a modification 4 of the first embodiment of the present invention;
Fig. 28 is a sectional view showing a state in which a globe-shaped expanded portion 22a12 of the blade distal end portion 22a comes into contact with the distal end portion 21d1 of the internal space 21d, the blade 22 is further pushed out to a distal end side, and the blade distal end portion 22a is bent according to the modification 4 of the first embodiment of the present invention;
Fig. 29 is a sectional view of the distal end portion of the puncture tool insertion portion 9A taken along line XXIX-XXIX in Fig. 28;
Fig. 30 is a configuration diagram showing a configuration of an operation system 1A using an ultrasound endoscope according to a modification 5 of the first embodiment of the present invention;
Fig. 31 is an external view of a puncture tool operation portion 9BX according to the modification 5 of the first embodiment of the present invention;
Fig. 32 is a sectional view of the puncture tool operation portion 9B taken along line XXXII-XXXII in Fig. 31;
Fig. 33 is a sectional view of a distal end portion of a puncture tool at a time of cutting a needle tube distal end portion 21b with a plane orthogonal to an axial direction of the needle tube 21, according to a second embodiment of the present invention;
Fig. 34 is a sectional view of a distal end portion of a modification of the puncture tool at a time of cutting the needle tube distal end portion 21b with a plane orthogonal to an axial direction of the needle tube 21 in a case where a blade portion is provided at two positions of the blade distal end portion 22a, according to the second embodiment of the present invention;
Fig. 35 is a sectional view of a puncture tool operation portion 9B1 along an axial direction according to the second embodiment of the present invention;
Fig. 36 is a plan view of a needle tube distal end portion 71 according to a third embodiment of the present invention;
Fig. 37 is a sectional view of the needle tube distal end portion 71 taken along line XXXVII-XXXVII in Fig. 36;
Fig. 38 is a perspective view of a blade distal end portion according to the third embodiment of the present invention;
Fig. 39 is a sectional view of the needle tube distal end portion 71 at a time when the blade distal end portion projects from an opening portion, according to the third embodiment of the present invention;
Fig. 40 is a sectional view along an axial direction of a needle tube distal end portion according to a modification of the third embodiment of the present invention;
Fig. 41 is a sectional view of the needle tube distal end portion along an axial direction of a needle tube at a time when the blade distal end portion in Fig. 40 projects from the opening portion;
Fig. 42 is a perspective view of a blade distal end portion 74 of the blade 22 seen from an upper surface side, according to the modification of the third embodiment of the present invention; and
Fig. 43 is a perspective view of the blade distal end portion 74 seen from a lower surface side, according to the modification of the third embodiment of the present invention.

### Best Mode for Carrying Out the Invention

Embodiments of the present invention are explained below with reference to the drawings.

Note that, in the respective drawings used for the following explanation, a scale is varied for each of components in order to show respective components in sizes enough for being recognized on the drawings. The present invention is not limited only to numbers of the components, shapes of the components, ratios of sizes of the components, and relative positional relations among the respective components shown in the drawings.

### (First Embodiment)

### (Configuration of Operation System)

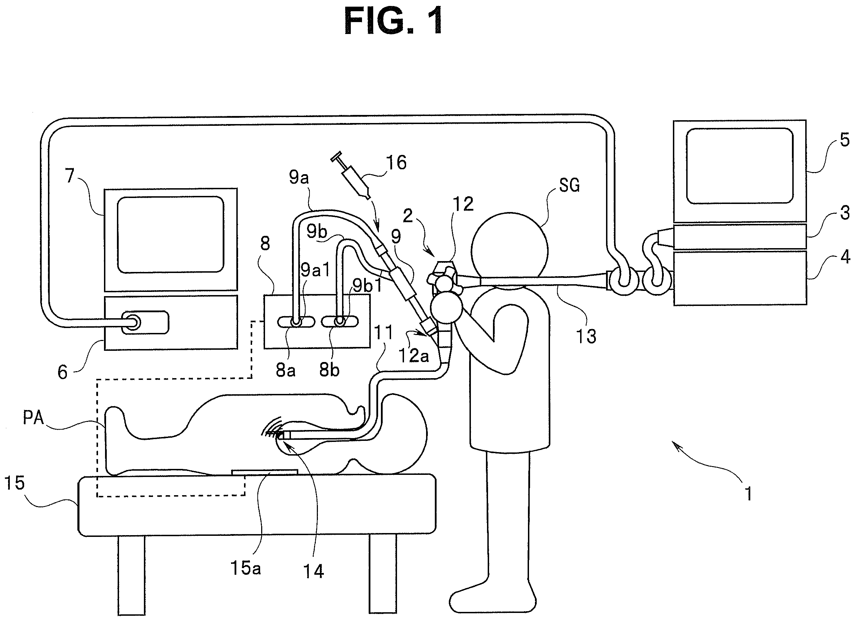

Fig. 1 is a configuration diagram showing a configuration of an operation system using an ultrasound endoscope according to the present first embodiment.

As shown in Fig. 1, an operation system 1 is configured with an ultrasound endoscope 2, a video processor 3, a light source device 4, a monitor 5, an ultrasound observation device 6, a monitor 7, a high frequency power supply device 8, and a puncture tool 9 which is a treatment instrument.

The ultrasound endoscope 2 is configured with an elongated endoscope insertion portion 11, an endoscope operation portion 12 and a universal cord 13. A distal end rigid portion 14 is provided at a distal end portion of the endoscope insertion portion 11. A treatment instrument insert-through port 12a is provided on a distal end side of the endoscope operation portion 12, and the ultrasound endoscope 2 is configured such that the puncture tool 9 can be attached to the treatment instrument insert-through port 12a. The ultrasound endoscope and the puncture tool 9, however, may be integrated.

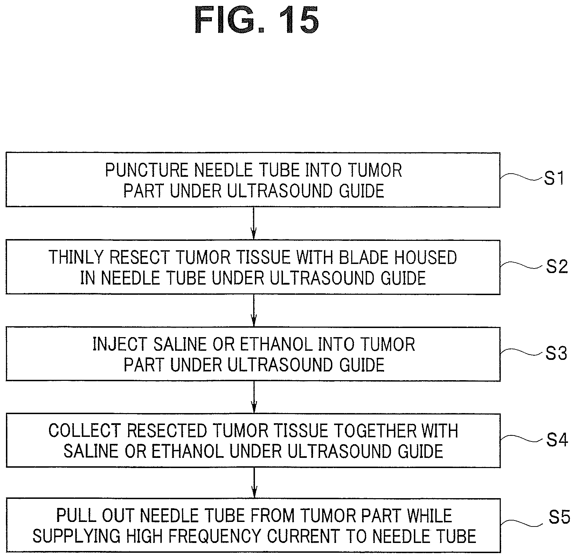

The distal end rigid portion 14 is provided with an illumination window for causing an illuminating light from the light source device 4 to exit, an observation window, an image pickup device provided on a backside of the observation window, and an ultrasound vibrating portion 14a (Fig. 15) though they are not shown. That is, the ultrasound endoscope 2 has the ultrasound vibrating portion 14a as an ultrasound observation portion which transmits ultrasound toward the puncture tool 9 and receives the ultrasound reflected from the puncture tool 9.

A surgeon SG inserts the endoscope insertion portion 11 through a mouth of a patient PA on a bed 15, grasping the endoscope operation portion 12 of the ultrasound endoscope 2 with one hand and grasping the endoscope insertion portion 11 with the other hand. An image of an inside of a body illuminated with an illuminating light from the light source device 4 is picked up by the image pickup device at the distal end portion of the endoscope insertion portion 11. An image pickup signal from the image pickup device is image-processed by the video processor 3, and an endoscopic image is displayed on the monitor 5. An ultrasound video signal obtained by the ultrasound vibrating portion at the distal end portion of the endoscope insertion portion 11 is image-processed by the ultrasound observation device 6, and an ultrasound image is displayed on the monitor 7.

Thus, the surgeon SG can not only perform an operation of inserting the endoscope insertion portion 11 and the like while viewing the endoscopic optical image displayed on the monitor 5 but also perform treatment of a lesion part using the puncture tool 9 while viewing the ultrasound image displayed on the monitor 7.

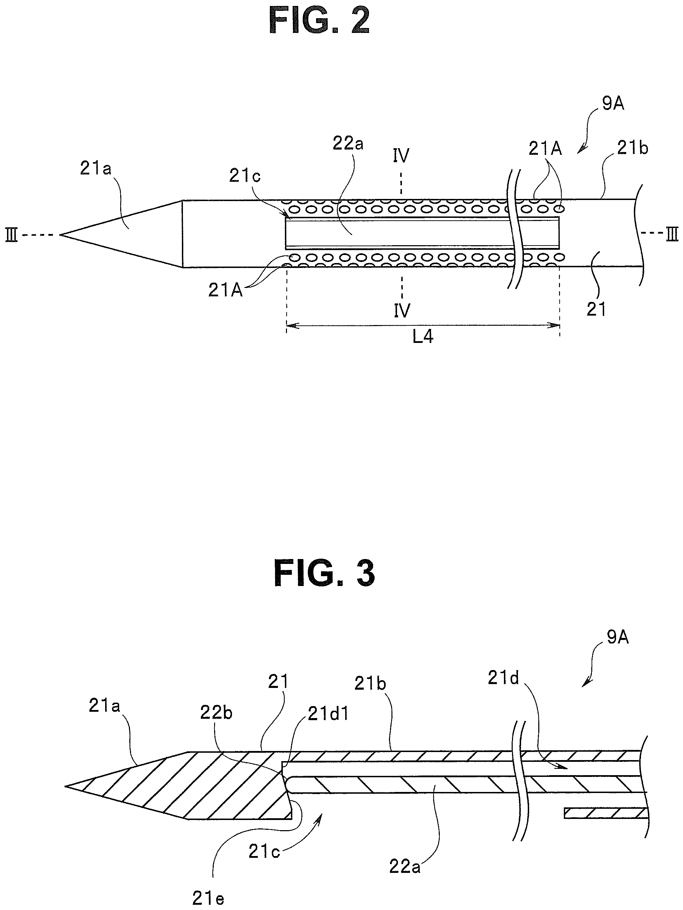

As explained later, the puncture tool 9 is capable of performing treatment using a high frequency current and capable of causing a high frequency current to flow through a needle tube 21 at a time of pulling out the needle tube 21 (Fig. 2) of the puncture tool 9 from a biological tissue to prevent adhesion of living lesion cells. The puncture tool 9 has two cables 9a and 9b and two connectors 9a1 and 9b1 provided at end parts of the two cables 9a and 9b.

For a time of causing a high frequency current to flow through the puncture tool 9, a counter electrode plate 15a to be in contact with the patient PA is placed on the bed 15.

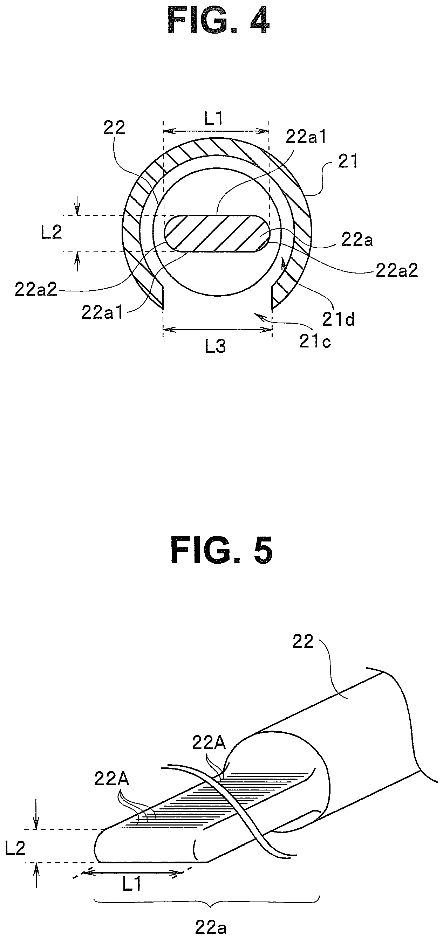

The high frequency power supply device 8 has two connectors 8a and 8b. The connector 9a1 is connected to the connector 8a, and the connector 9b1 is connected to the connector 8b. A high frequency current is supplied to a blade 22 (Fig. 5) of the puncture tool 9 via the cable 9a, and a high frequency current is supplied to the needle tube 21 (Fig. 2) of the puncture tool 9 via the cable 9b.

Supply of the high frequency current to each of the blade 22 and the needle tube 21 can be performed by the surgeon operating a switch (not shown) provided on the high frequency power supply device 8.

As explained later, a syringe 16 can be attached to the puncture tool 9. As explained later, the syringe 16 is a suction device capable of injecting liquid into the needle tube 21 and sucking liquid or the like from the inside of the needle tube 21.

### (Configuration of Puncture Tool)

The puncture tool 9 includes an elongated puncture tool insertion portion 9A, through which the needle tube 21 and the blade 22 are inserted, and a puncture tool operation portion 9B provided on a proximal end side of the puncture tool insertion portion 9A and for performing projecting operation for the needle tube 21 and the like. The puncture tool 9 is configured such that the puncture tool insertion portion 9A can be inserted from the treatment instrument insert-through port 12a of the endoscope operation portion 12 and a distal end portion of the puncture tool insertion portion 9A can be projected from the treatment instrument opening of the distal end rigid portion 14 through the treatment instrument insert-through channel in the endoscope insertion portion 11.

First, a configuration of the puncture tool insertion portion 9A is explained. The puncture tool insertion portion 9A includes the sheath 41 and the needle tube 21 and the blade 22 inserted through the sheath 41.

Fig. 2 is a plan view of the distal end portion of the puncture tool insertion portion 9A. Fig. 3 is a sectional view of the distal end portion of the puncture tool insertion portion 9A taken along line III-III in Fig. 2. Fig. 4 is a sectional view of the distal end portion of the puncture tool insertion portion 9A taken along line IV-IV in Fig. 2. Fig. 5 is a perspective view of a distal end portion of the blade 22.

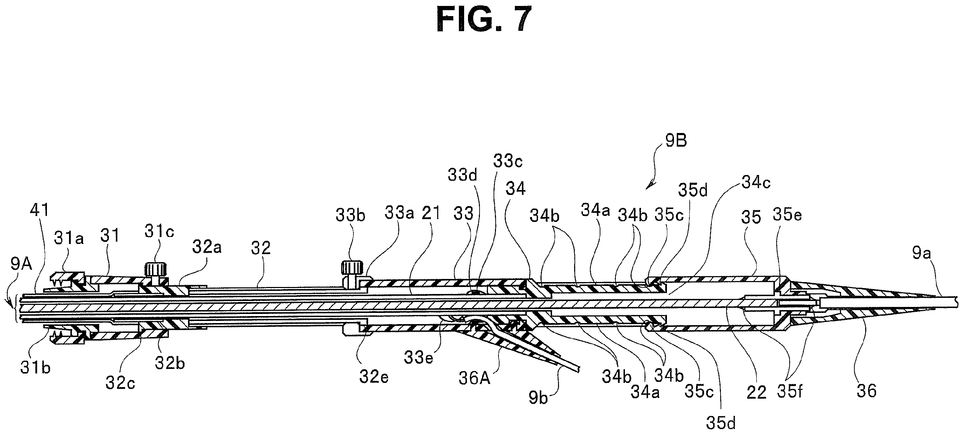

The puncture tool insertion portion 9A includes the sheath 41 (Fig. 7) not shown in Fig. 2, the needle tube 21 inserted through the sheath 41, and the blade 22 inserted through the needle tube 21.

As shown in Figs. 2 and 3, the needle tube 21 is a tubular member of stainless steel, nickel titanium, a cobalt chrome alloy, or the like, a hole at a distal end portion of which is closed by welding or the like. A tubular portion internally including a channel whose distal end side opens on a side surface is configured in the needle tube 21. The hollow and elongated needle tube 21 includes a needle tube distal end portion 21b including a sharp conical shape portion 21 a at a distal end. The needle tube distal end portion 21b includes an elongated opening portion 21c formed along an axial direction. The opening portion 21c is provided in a vicinity of the conical shape portion 21a. That is, the opening portion 21c is provided on a proximal end side of a distal end portion of the needle tube distal end portion 21b and communicates with the channel in the needle tube 21. The conical shape portion 21a is arranged at the distal end surface of the needle tube 21, which is the tubular portion, and configures a puncture portion to be punctured into a subject. A diameter of the needle tube 21 is, for example, 22G to 19G (gauge). Length in an axial direction of the opening portion 21c is, for example, 5 to 15 mm. That is, a distal end of the needle tube 21 has a so-called pencil shape.

The blade 22, which is an elongated shaft member having a circular section, is inserted through the internal space 21d of a hollow needle tube distal end portion 21b. A diameter of a shaft portion of the blade 22 is, for example, 0.5 to 1 mm. The blade 22 is also made of metal such as stainless steel, nickel titanium, or a cobalt chrome alloy.

Electrical resistance of the needle tube 21 is higher than that of a cutting portion to be explained later. Thereby, switching between energization of the needle tube 21 and energization of the cutting portion can be performed by insertion/pulling out of the blade 22 to/from the channel. By setting the electrical resistance of the needle tube 21 higher than that of the cutting portion, most of a current flows to a cutting portion side with a lower resistance even if the cutting portion and the needle tube 21 are energized simultaneously. If energization is continued from a time of cutting to a time of pulling out, a current is concentrated to the cutting portion at the time of cutting by the cutting portion. After that, by pulling out the blade 22 from the channel, the current flows only through the needle tube 21, and the needle tube 21 is energized at the time of pulling out.

As an example of realizing the difference between the electrical resistances, means of manufacturing the needle tube 21 and the blade 22 with different materials is conceivable.

Processing for oxidizing a surface to increase contact resistance and reduce electric conductivity with electric discharge processing or processing for forming an insulating film by surface coating using polyimide or the like is applied to a channel inner side surface of the needle tube distal end portion 21b.

Alternatively, the processing for increasing the contact resistance or the processing for forming the insulating film may be applied to a surface of the blade 22 in a portion other than a portion in contact with a distal end side inner wall portion 21e at the blade distal end portion 22a and a portion projecting from the opening portion 21c of the blade distal end portion 22a. Note that, in this case, the processing for increasing the contact resistance or the processing for forming the insulating film may be applied to a channel inner side surface of the needle tube 21 as well.

As explained above, the processing for reducing electric conductivity between the blade 22 and an inner wall of the needle tube distal end portion 21b or the needle tube 21 having the puncture portion or the processing for performing electric insulation between the blade 22 and the inner wall of the needle tube distal end portion 21b or the needle tube 21 is applied to at least one of the blade 22 and the needle tube 21.

As shown in Fig. 4, a sectional shape in a direction orthogonal to an axial direction of the blade distal end portion 22a of the blade 22 includes two plane portions 22a1 and semicircular portions 22a2 at both ends of the two plane portions 22a1. That is, the blade distal end portion 22a includes a flat portion having a flat section and a thin extending tabular shape. Width L1 of the tabular shape is, for example, 0.3 to 0.9 mm. Thickness L2 of the tabular shape is, for example, 0.1 to 0.2 mm.

As shown in Fig. 3, a curved surface portion 22b semicircular in a sectional shape extending along the axial direction of the blade 22 is provided at a distal end of the blade distal end portion 22a. Further, ultrasound reflection processing for reflecting ultrasound is applied to the plane portions 22a1. As shown in Fig. 5, a large number of fine grooves 22A are formed as ultrasound reflection processing portions in the two plane portions 22a1.

Note that, as explained below, the large number of grooves 22A of the ultrasound reflection processing portions may have a shape formed in a range in which the blade distal end portion 22a bends in an arcuate shape and formed along a direction orthogonal to the axial direction of the blade distal end portion 22a and contributing to bending of the blade distal end portion 22a.

The width L1 of the blade distal end portion 22a is smaller than width L3 of the opening portion 21c of the needle tube 21. As explained below, the blade distal end portion 22a and the opening portion 21c are configured such that, when the blade distal end portion 22a bends, a bending portion of the blade distal end portion 22a can project from the opening portion 21c. The bending portion formed when the blade distal end portion 22a projects from the opening portion 21c configures a cutting portion. That is, the cutting portion is a bending portion which is exposed from the opening portion 21c in a state of being bent by the distal end of the blade distal end portion 22a coming into contact with a distal end of the opening portion 21c when the blade 22 projects from the opening portion 21c.

As explained above, the blade 22 can be inserted through the channel of the needle tube 21. The blade 22 is configured such that, when the blade 22 is inserted through the channel, the bending portion functioning as the cutting portion can project from the opening portion 21c.

As shown in Fig. 2, the ultrasound reflection processing is applied to the needle tube distal end portion 21 b over a predetermined range from a distal end portion to a proximal end portion of the opening portion 21c. Specifically, dimple processing of a plurality of dimples 21 A is applied as ultrasound reflection processing portions over a range L4 in which the opening portion 21c is present on an outer side surface of the needle tube distal end portion 21b.

Note that, although the ultrasound reflection processing portions of the plurality of dimples 21A are formed over the range L4 in which the opening portion 21c is present, the ultrasound reflection processing portions may be provided only in two places of the distal end portion of the opening portion 21c and the proximal end portion of the opening portion 21 c such that the dimple processing is absent between the distal end portion and the proximal end portion of the opening portion 21 c.

The distal end side inner wall portion 21e of the needle tube distal end portion 21 b forming the internal space 21 d has a slope inclined at a predetermined angle with respect to a surface orthogonal to an axial direction of the needle tube 21. A wall surface of the distal end side inner wall portion 21 e is formed as a slope extending closer to a proximal end direction of the needle tube 21 from the distal end portion 21d1 of the internal space 21d toward the opening portion 21c. In other words, the wall surface of the distal end side inner wall portion 21e is formed as a slope extending closer to the distal end portion of the needle tube 21 from the opening portion 21 c toward depth of the internal space 21d. Therefore, the needle tube distal end portion 21 b has such an inclined surface that a section on a puncture portion side of the opening portion 21c forms an acute angle to prevent the blade distal end portion 22a from projecting from the opening portion 21c when the bending portion of the blade distal end portion 22a is formed.

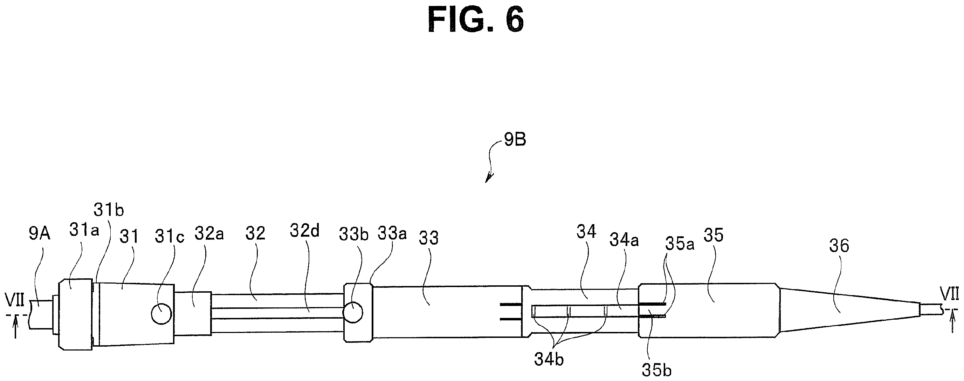

Next, a configuration of the puncture tool operation portion 9B connected to a proximal end portion of the puncture tool insertion portion 9A is explained. Fig. 6 is an external view of the puncture tool operation portion 9B. Fig. 7 is a sectional view of the puncture tool operation portion 9B taken along line VII-VII of Fig. 6.

The puncture tool operation portion 9B is attached and fixed to the treatment instrument insert-through port 12a of the ultrasound endoscope 2. A connecting portion 31 for attachment to the treatment instrument insert-through port 12a is provided at a distal end portion of the puncture tool operation portion 9B. Further, the puncture tool operation portion 9B includes a main body 32, a needle tube slider 33, a needle tube turning operation portion 34, a blade slider 35, and a bend preventing portion 36 for protecting a cable 9a in order toward a proximal end side.

The connecting portion 31 includes a connection ring 31 a and a distal end connecting member 31b on a distal end side. The connecting portion 31 is attached to the treatment instrument insert-through port 12a of the endoscope operation portion 12 by internally inserting the distal end connecting member 31 b into the treatment instrument insert-through port 12a and the connection ring 31a is turned in a predetermined direction, whereby the puncture tool operation portion 9B can be fixed to the endoscope operation portion 12. A sheath fixing knob 31c is provided on a proximal end side of the connecting portion 31.

A connecting member 32a is provided on a distal end side of the main body 32. The sheath 41 of the puncture tool insertion portion 9A is externally inserted over and fixed to a connection pipe 32b provided at a distal end of the connecting member 32a. The connecting member 32a is loosely fit in the connecting portion 31 having a cylindrical shape. The sheath 41 can be fixed to the connecting portion 31 in a desired position by turning the sheath fixing knob 31c in a predetermined direction. A stopper 32c that comes into contact with an inner peripheral side convex portion of the cylindrical connecting portion 31 to prevent the connecting member 32a from coming off the connecting portion 31 is provided on a distal end side of the connecting member 32a.

A main body groove portion 32d is formed along an axial direction of the main body 32 on an outer surface of the main body 32. A connecting member 33a is provided on a distal end side of the needle tube slider 33. A stopper 32e that comes into contact with an inner peripheral side convex portion of a cylindrical connecting member 33a to prevent the main body 32 from coming off the needle tube slider 33 is provided on a proximal end side of the main body 32.

A needle tube fixing knob 33b is provided in the connecting member 33a. On a proximal end side, the main body 32 is loosely fit in the needle tube slider 33 having a cylindrical shape. The needle tube slider 33 can be fixed to the main body 32 in a desired position by turning the needle tube fixing knob 33b in a predetermined direction.

In a proximal end portion of the needle tube slider 33, the needle tube turning operation portion 34 having a cylindrical shape is provided to be engaged with the needle tube slider 33 to be capable of turning around an axis of the needle tube turning operation portion 34. Two blade adjusting grooves 34a are provided along an axial direction of the needle tube turning operation portion 34 on an outer surface of the needle tube turning operation portion 34. Further, a plurality of (four here) concave portions 34b are provided at a predetermined interval along an axial direction in the respective blade adjusting grooves 34a.

The needle tube 21 is inserted through the sheath 41 of the puncture tool insertion portion 9A. The blade 22 is inserted through the needle tube 21. A proximal end of the needle tube 21 is fixed to a distal end portion of the needle tube turning operation portion 34. Therefore, when the needle tube turning operation portion 34 is turned around an axis, the needle tube 21 also turns around an axis.

The blade slider 35 having a cylindrical shape is externally inserted over and provided on a proximal end side of the needle tube turning operation portion 34. On a distal end side of the blade slider 35, two engaging portions 35b held between two grooves 35a formed along an axial direction are provided. Two convex portions 35c projecting to an inner side and stoppers 35d projecting to the inner side are provided in the engaging portion 35b. The two convex portions 35c engage with the two blade adjusting grooves 34a. The respective convex portions 35c are formed in the blade slider 35 to press outer side surfaces of the blade adjusting grooves 34a and to be movable along the axial direction of the needle tube turning operation portion 34.

A cylindrical blade fixing cap 35e is fixed and provided on the distal end side of the blade slider 35. A proximal end portion of the blade 22 is inserted from a distal end side of the blade fixing cap 35e. A signal line of the cable 9a is inserted from a rear end side of the blade fixing cap 35e. The blade 22 and the cable 9a are soldered and fixed by solder 35f. Therefore, the blade 22 is fixed to the blade slider 35 and electrically connected to the cable 9a by the blade fixing cap 35e. That is, the cable 9a connected to the blade 22 and the connector 9a1 configure a power supply connecting portion for electrically connecting the blade distal end portion 22a, which is the cutting portion of the blade 22, and the high frequency power supply device 8, which is a power supply.

Therefore, when the surgeon moves the blade slider 35 along the axial direction of the needle tube turning operation portion 34, the blade slider 35 is lightly fixed to the needle tube turning operation portion 34 in a position where the convex portions 35c engage with the concave portions 34b. However, the blade slider 35 can be moved along the axial direction of the needle tube turning operation portion 34 with a stronger force. The stopper 35d is a stopper that comes into contact with an outer peripheral side convex portion of the needle tube turning operation portion 34 to prevent the blade slider 35 from coming off the needle tube turning operation portion 34.

Further, when the blade slider 35 is moved to a proximal end side to separate the engaging portion 35b from a surface of the needle tube turning operation portion 34 and cause the stopper 35d to climb over the outer peripheral side convex portion of the needle tube turning operation portion 34, the blade slider 35 can be detached from the needle tube turning operation portion 34 and the blade 22 can be pulled out from the needle tube 21.

An opening portion 34c of a proximal end portion of the needle tube turning operation portion 34, which is exposed when the blade slider 35 is pulled out, is provided with a slight taper (lure taper) so that the syringe 16 can be attached. That is, the opening portion 34c at the proximal end portion of the needle tube turning operation portion 34 configures a suction device connecting portion for connecting the syringe 16, which is a suction device arranged on a proximal end side of the channel of the needle tube 21.

The cable 9b extends from a side surface portion of the needle tube slider 33. A bend preventing portion 36A is provided in the needle tube slider 33 to protect surroundings of the cable 9b. A distal end portion of the cable 9b is pressed against the needle tube 21 by a binder 33c, which is a fixing member. The distal end portion of the cable 9b is fixed to the needle tube 21 by applying an adhesive 33d around the binder 33c. The signal line of the cable 9b is soldered to the needle tube 21 by solder 33e on an inner portion of the needle tube slider 33. As explained above, the connector 9b1 for connecting the cable 9b to the high frequency power supply device 8 is provided at a proximal end portion of the cable 9b. Therefore, the cable 9b connected to the needle tube distal end portion 21b including the puncture portion and the connector 9b1 configure a power supply connecting portion for electrically connecting the needle tube distal end portion 21b including the conical shape portion 21 a, which is the puncture portion, and the high frequency power supply device 8, which is the power supply.

Since the puncture tool operation portion 9B has the configuration explained above, the surgeon can project and retract, from the treatment instrument opening of the distal end rigid portion 14 of the endoscope insertion portion 11, each of the sheath 41, the needle tube 21, and the blade 22 of the puncture tool insertion portion 9A inserted through the treatment instrument insert-through channel of the ultrasound endoscope 2 by operating the puncture tool operation portion 9B.

Fig. 6 and Fig. 7 show a state in which the sheath 41, the needle tube 21, and the blade 22 are drawn into a most proximal end side of the puncture tool insertion portion 9A. When the puncture tool 9 is attached to the treatment instrument insert-through port 12a, in the state shown in Fig. 6 and Fig. 7, the sheath 41, the needle tube 21, and the blade 22 do not project from the treatment instrument opening of the distal end rigid portion 14 of the endoscope insertion portion 11.

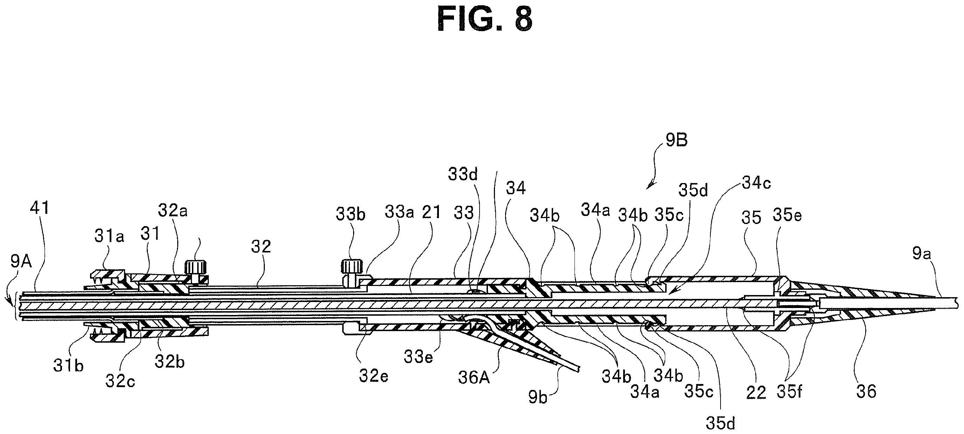

The surgeon SG can change the puncture tool operation portion 9B from the state shown in Fig. 6 and Fig. 7 to a state shown in Fig. 8 by moving the main body 32 to the distal end side with respect to the connecting portion 31. Fig. 8 is a sectional view of the puncture tool operation portion 9B in a state in which the sheath 41 projects most from the treatment instrument opening of the distal end rigid portion 14.

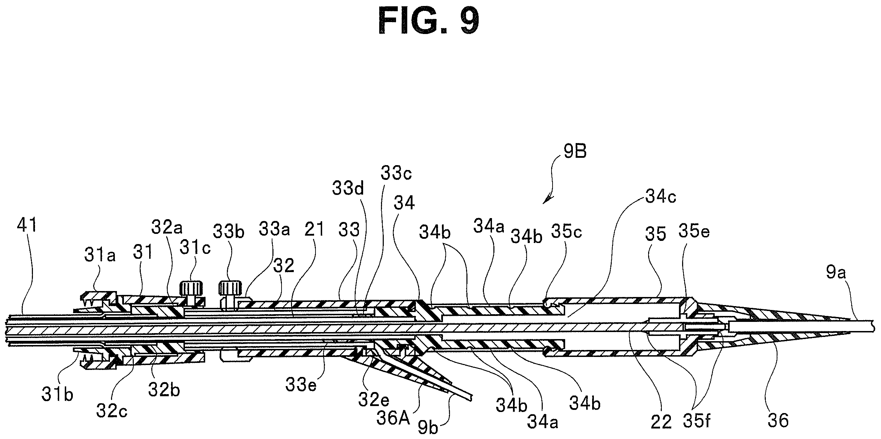

The surgeon SG can change the puncture tool operation portion 9B from the state shown in Fig. 8 to a state shown in Fig. 9 by moving the needle tube slider 33 to the distal end side with respect to the main body 32. Fig. 9 is a sectional view of the puncture tool operation portion 9B in a state in which the sheath 41 and the needle tube 21 project most from the treatment instrument opening of the distal end rigid portion 14.

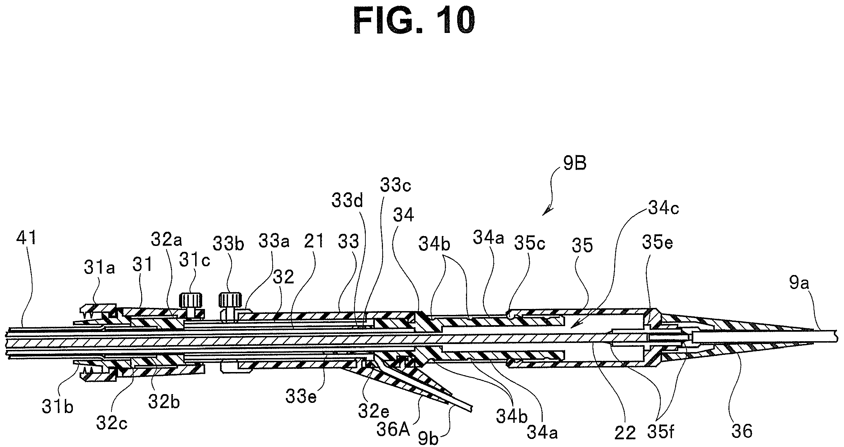

The surgeon SG can change the puncture tool operation portion 9B from the state shown in Fig. 9 to a state shown in Fig. 10 by moving the blade slider 35 to the distal end side with respect to the needle tube turning operation portion 34. Fig. 10 is a sectional view of the puncture tool operation portion 9B in a state in which the sheath 41 and the needle tube 21 project most from the treatment instrument opening of the distal end rigid portion 14 and the blade 22 is projected by one stage from the treatment instrument opening of the distal end rigid portion 14.

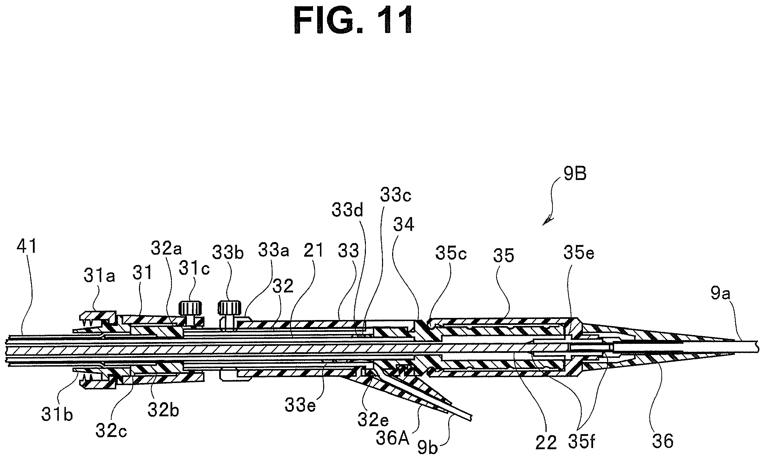

The surgeon SG can change the puncture tool operation portion 9B from the state shown in Fig. 10 to a state shown in Fig. 11 by further moving the blade slider 35 to the distal end side with respect to the needle tube turning operation portion 34. Fig. 11 is a sectional view of the puncture tool operation portion 9B in a state in which the sheath 41 and the needle tube 21 project most from the treatment instrument opening of the distal end rigid portion 14 and the blade 22 is projected most from the treatment instrument opening of the distal end rigid portion 14.

When the puncture tool operation portion 9B is in the state shown in Fig. 10, the blade distal end portion 22a bends and the bending portion of the blade distal end portion 22a projects from the opening portion 21c. When the puncture tool operation portion 9B is in the state shown in Fig. 11, the blade distal end portion 22a further bends and the bending portion, which is the cutting portion, further projects from the opening portion 21 c.

Therefore, the surgeon can project each of the sheath 41, the needle tube 21, and the blade 22 at the distal end portion of the puncture tool insertion portion 9A by a desired amount from the treatment instrument opening of the distal end rigid portion 14 and retract each of the sheath 41, the needle tube 21, and the blade 22 into the treatment instrument opening of the distal end rigid portion 14 by operating the respective portions of the puncture tool operation portion 9B.

When the blade 22 is pushed out to the distal end side, the curved surface portion 22b semicircular in a sectional shape comes into contact with the distal end side inner wall portion 21 e. When the blade 22 is further pushed out to the distal end side, the curved surface portion 22b moves along the slope of the distal end side inner wall portion 21e and comes into contact with the distal end portion 21d1 of the internal space 21d. Therefore, the blade distal end portion 22a bends to project from the opening portion 21c.

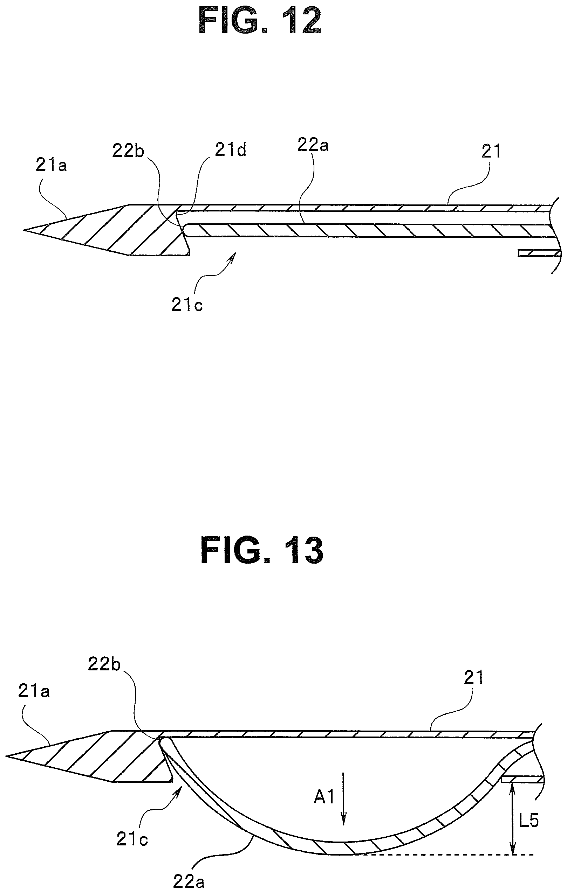

Fig. 12 is a sectional view showing a state in which the blade distal end portion 22a comes into contact with the distal end portion 21d1 of the internal space 21d but the blade distal end portion 22a is not bent. Fig. 13 is a sectional view showing a state in which the blade distal end portion 22a comes into contact with the distal end portion 21d1 of the internal space 21d and the blade 22 is further pushed out to the distal end side and the blade distal end portion 22a is bent.

A projection amount L5 of the blade distal end portion 22a changes according to a movement amount of the blade slider 35 to the distal end side with respect to the needle tube turning operation portion 34. Respective positions of the concave portions 34b correspond to projection amounts of the blade distal end portion 22a. Therefore, the surgeon can change the projection amount L5 of the blade distal end portion 22a by moving the blade slider 35 along the axial direction of the needle tube turning operation portion 34 and changing the positions of the concave portions 34b of the needle tube turning operation portion 34 with which the respective convex portions 35c of the blade slider 35 engage. As the surgeon moves the blade slider 35 further to the distal end side with respect to the needle tube turning operation portion 34, the blade distal end portion 22a projects further in a direction indicated by an arrow A1 and the projection amount L5 of the blade distal end portion 22a from the opening portion 21 c increases.

### (Operation)

Treatment using the puncture tool 9 explained above is explained. Here, an example of a manipulation for reducing a lesion part of pancreatic cancer is explained.

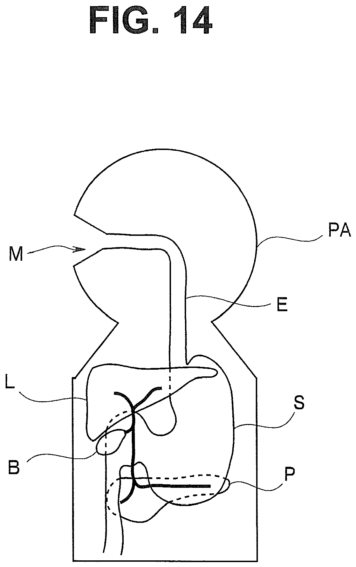

Fig. 14 is a schematic explanatory diagram of a human body including a pancreas and a stomach. The surgeon inserts the endoscope insertion portion 11 down to a stomach S from a mouth M of the human body through an esophagus E. A pancreas P exists in a vicinity of the stomach S. A liver L and a gallbladder B exist around the stomach S.

Fig. 15 is a diagram for explaining a flow of a manipulation for reducing a tumor part Pa, which is a lesion part of the pancreas P. First, as shown in Fig. 1, the surgeon SG inserts the distal end portion of the endoscope insertion portion 11 from the mouth M of the patient PA through the esophagus E.

The surgeon SG brings the ultrasound vibrating portion 14a at the distal end portion of the endoscope insertion portion 11 into close contact with a stomach wall of the stomach S and punctures the needle tube 21 into the tumor part Pa under an ultrasound guide (S1). That is, the surgeon SG can operate the main body 32 and the needle tube slider 33 of the puncture tool operation portion 9B while viewing an ultrasound image displayed on the monitor 7 to cause the needle tube 21 to project and puncture the needle tube 21 into the tumor part Pa of the pancreas P.

Subsequently, the surgeon thinly resects a tumor tissue with the blade 22 housed in the needle tube 21 under the ultrasound guide (S2).

For example, the surgeon can resect the tumor tissue by projecting the blade distal end portion 22a while viewing an ultrasound image displayed on the monitor 7 and checking a projecting state of the bending portion of the blade distal end portion 22a and by turning the needle tube 21 little by little around the axis while supplying a high frequency current to the blade 22.

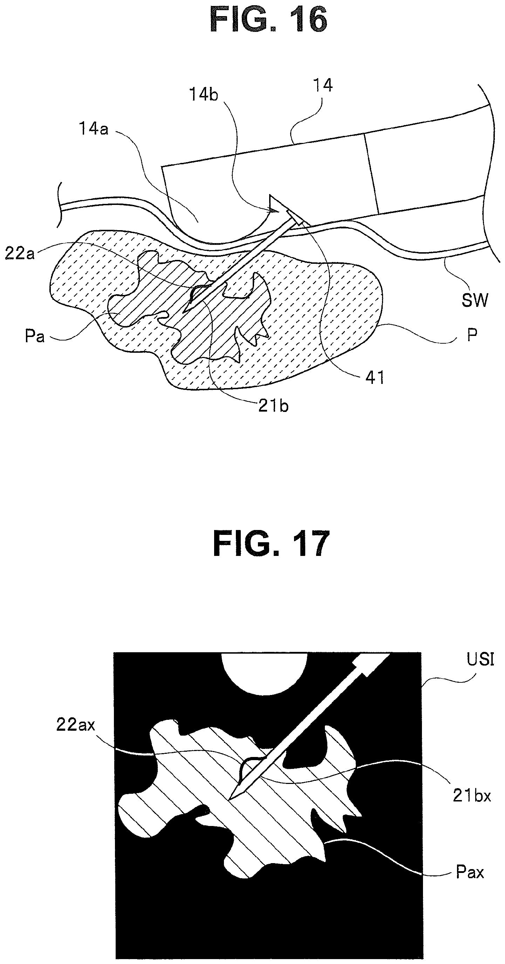

Specifically, the surgeon SG can project the blade distal end portion 22a from the opening portion 21c by moving the blade slider 35 to the distal end side. Fig. 16 is a diagram for explaining a projecting state of the blade distal end portion 22a. As shown in Fig. 16, the needle tube distal end portion 21b pierces through the stomach wall SW and enters into the tumor part Pa of the pancreas P. At this point, as explained above, the projection amount L5 of the bent blade distal end portion 22a from the opening portion 21c can be adjusted according to a movement amount of the blade slider 35 with respect to the needle tube turning operation portion 34.

Fig. 17 is a diagram showing an example of an ultrasound image displayed on the monitor 7. The ultrasound image USI obtained by the ultrasound vibrating portion 14a is displayed on the display screen of the monitor 7. An image 21bx of the needle tube distal end portion 21b and an image 22ax of the blade distal end portion 22a are clearly displayed on the ultrasound image USI by the ultrasound reflection processing portions provided on the surfaces of the needle tube distal end portion 21b and the blade distal end portion 22a. The image Pax of the tumor part Pa is also displayed on the ultrasound image USI.

Therefore, the surgeon SG can grasp a position of the needle tube distal end portion 21b and a projection amount of the blade distal end portion 22a while viewing the ultrasound image USI.

The surgeon SG can resect the tumor tissue of the tumor part Pa with the bending portion of the blade distal end portion 22a, which is the cutting portion, by locating the projected blade distal end portion 22a in a position where the inner portion of the tumor part Pa can be shaved off and turning the needle tube 21 around the axis while feeding a high frequency current to the blade 22 under the ultrasound guide.

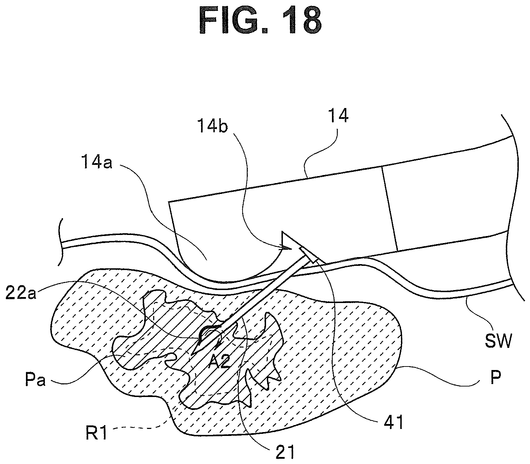

Fig. 18 is a diagram for explaining a movement of the blade distal end portion 22a. As shown in Fig. 18, the surgeon moves the needle tube 21 in the inner portion of the tumor part Pa as indicated by a dotted line R1 while turning the needle tube 21 around the axis as indicated by an arrow A2. At this point, if the blade distal end portion 22a is turned as indicated by the arrow A2, since the high frequency current is flowing to the blade 22, the bending portion of the blade distal end portion 22a can shave off the tumor tissue and resect the tumor tissue into small tumor tissue pieces in the same manner as an electric knife. If the needle tube 21 is moved in the inner portion of the tumor part Pa as indicated by the dotted line R1 while turning the needle tube 21 around the axis, one tumor part Pa is decomposed into a large number of or a plurality of fine tumor tissue pieces. The surgeon can determine, viewing the ultrasound image USI, a degree of the resection of the tumor tissue of the tumor part Pa, that is, to which degree the tumor tissue is resected.

It is preferable that the resection of the tumor tissue is performed to leave only a thin portion on the outer side surface of the tumor part Pa. That is, the surgeon resects the tumor tissue pieces while viewing an ultrasound image until the entire tumor tissue is resected to leave a layer of an outer contour of the tumor part Pa without cutting a normal biological tissue of the pancreas P around the tumor part Pa. Note that a range to be resected may be only a desired region of the tumor part Pa.

Subsequently, the surgeon SG injects saline or ethanol into the tumor part Pa through the needle tube distal end portion 21b under the ultrasound guide (S3).

The surgeon pours saline or ethanol into the syringe main body of the syringe 16 in advance. After S2, as explained above, the surgeon SG detaches the blade slider 35 of the puncture tool 9 from the needle tube turning operation portion 34, pulls out the blade 22 from the needle tube 21, attaches the syringe 16 including the saline or the ethanol to the opening portion 34c of the proximal end portion of the needle tube turning operation portion 34, from which the blade slider 35 is pulled out, and pushes in the plunger of the syringe 16 to thereby perform injection of the saline or the ethanol.

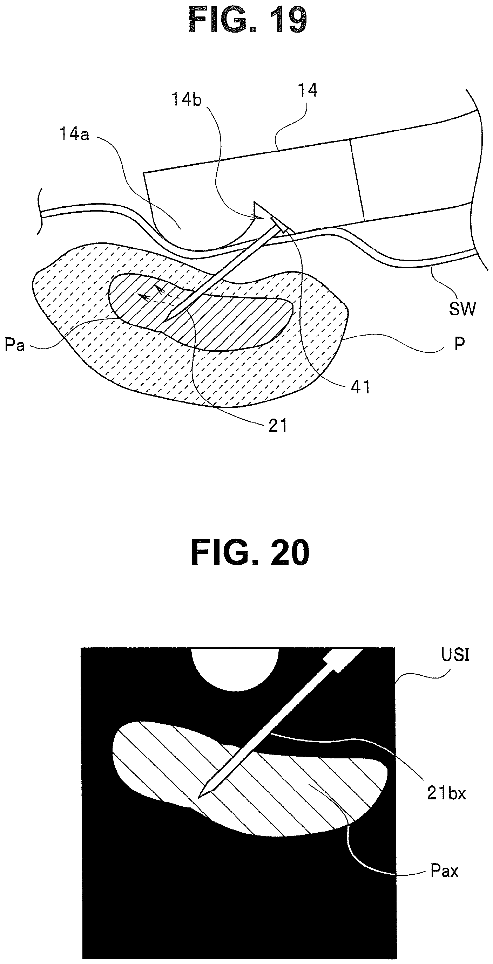

Fig. 19 is a diagram for explaining the injection of the saline or the ethanol. When the surgeon SG injects the saline or the ethanol from the syringe 16, as indicated by a dotted line in Fig. 19, the saline or the ethanol is injected into an inner portion of the layer of the outer contour of the tumor part Pa from the opening portion 21c. Therefore, the tumor part Pa bulges and a volume of the tumor part Pa increases. The surgeon SG can view a situation of the injection of the saline or the ethanol using an ultrasound image displayed on the monitor 7.

Fig. 20 is a diagram showing an example of the ultrasound image displayed on the monitor 7 when the saline or the ethanol is injected. As shown in Fig. 20, the image Pax of the bulged tumor part Pa is displayed on the monitor 7. Therefore, the surgeon SG can inject the saline or the ethanol into the tumor part Pa by a desired amount while viewing the ultrasound image.

The surgeon SG collects the resected tumor tissue pieces together with the saline or the ethanol under the ultrasound guide (S4). The collection of the resected tumor tissue pieces is performed by the operation of the syringe 16.

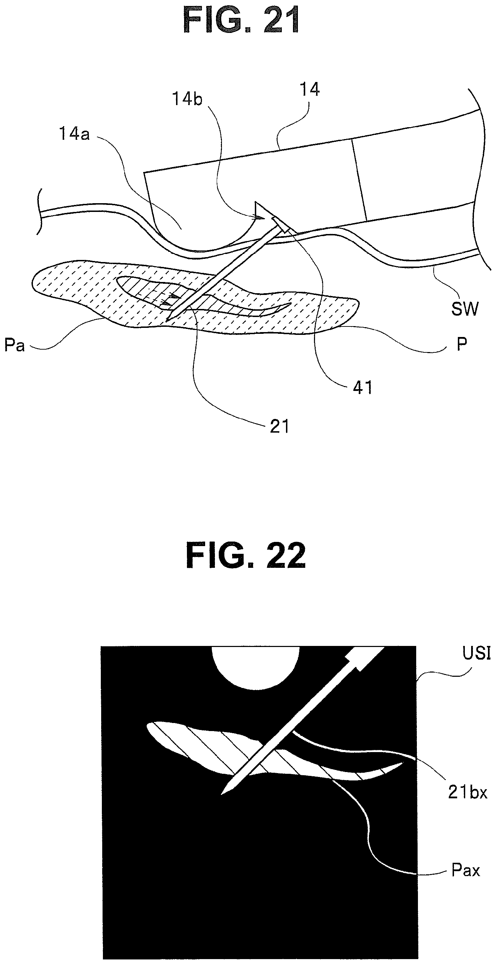

Fig. 21 is a diagram for explaining the collection of the resected tumor tissue pieces. When the surgeon SG operates the plunger of the syringe 16 to pull out the plunger from the syringe main body, as indicated by a dotted line in Fig. 21, the resected tumor tissue pieces are sucked from the opening portion 21c together with the saline or the ethanol and the volume of the tumor part Pa decreases. That is, when the saline or the ethanol is injected into the tumor part Pa in S3, fine fragments of the resected tumor tissue are included in the saline or the ethanol. Therefore, when the resected tumor tissue is sucked by the syringe 16 together with the saline or the ethanol, the size of the tumor part Pa decreases.

The surgeon SG can view a situation of the collection of the tumor tissue using the ultrasound image displayed on the monitor 7. Fig. 22 is a diagram showing an example of the ultrasound image displayed on the monitor 7 when the resected tumor tissue pieces are collected. As shown in Fig. 22, the image Pax of the reduced tumor part Pa is displayed on the monitor 7.

Further, the surgeon SG pulls out the needle tube 21 from the inside of the tumor part Pa while supplying a high frequency current to the needle tube 21 (S5). Since the high frequency current is flowing through the needle tube 21, a biological tissue in contact with an outer surface of the needle tube 21 is burned by the high frequency current and, therefore, adhesion of living lesion cells can be prevented.

As explained above, with the puncture tool of the above embodiment, since a resected tumor tissue in the tumor part Pa is sucked, and a size of the tumor part Pa of the pancreas P can be reduced, it is possible to reduce or eliminate oppression on peripheral organs, blood vessels, and nerves by the tumor part Pa. A pain due to oppression based on increase in a tumor is also reduced, and QOL (quality of life) is also improved.

Since the resected tumor tissue has been sucked, there is also an effect that tumor lysis syndromes due to remaining of a cancer cell or the like in the body are suppressed.

Further, since the size of the tumor part Pa is reduced, and the tumor part Pa is away from surrounding blood vessels and the like, a surgical operation becomes possible, and there may be a case the tumor part Pa can be excised.

Note that, though saline or ethanol is injected at S3, it is also possible to inject saline or ethanol after injecting trypsin which dissolves the resected tumor tissue. When the resected tumor tissue includes a lot of fiber, it is also possible to inject plasmin antagonist instead of saline or ethanol after injecting plasmin.

Further, note that, though the counter electrode plate 15a is used to cause a high frequency current to flow in the operation system 1 explained above, an operation system may adopt a bipolar configuration in which a current is caused to flow between the blade distal end portion 22a and the needle tube distal end portion 21b without using the counter electrode plate 15a.

### (Modification 1)

Though the distal end portion of the needle tube distal end portion 21b is in a conical shape in the embodiment explained above, the distal end portion may be in a trocar point shape.

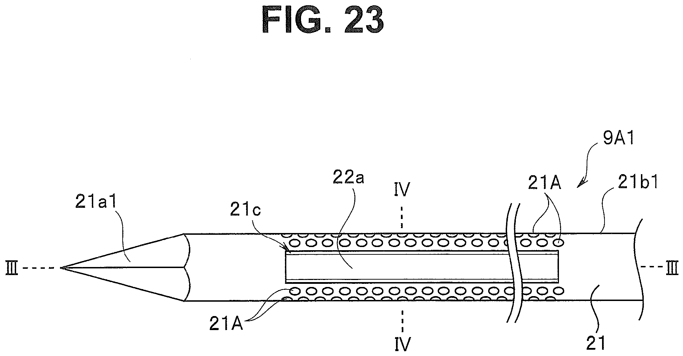

Fig. 23 is a plan view of a needle tube distal end portion 21b1 of a puncture tool insertion portion 9A1 according to the present modification 1. A sectional view of a distal end portion of the puncture tool insertion portion 9A taken along line III-III of the needle tube distal end portion 21b1 of the present modification 1 is same as Fig. 3. A sectional view of the distal end portion of the puncture tool insertion portion 9A1 taken along line IV-IV of the needle tube distal end portion 21b1 of the present modification 1 is same as Fig. 4.

Even though a distal end portion of the needle tube distal end portion 21b1 of the present modification 1 is such a trocar point shape portion 21a1, effects similar to the effects of the puncture tool in the embodiment explained above are obtained.

### (Modification 2)

Though the distal end portion of the needle tube distal end portion 21 b is in a conical shape in the embodiment explained above, the distal end portion may be in a bevel-cut shape.

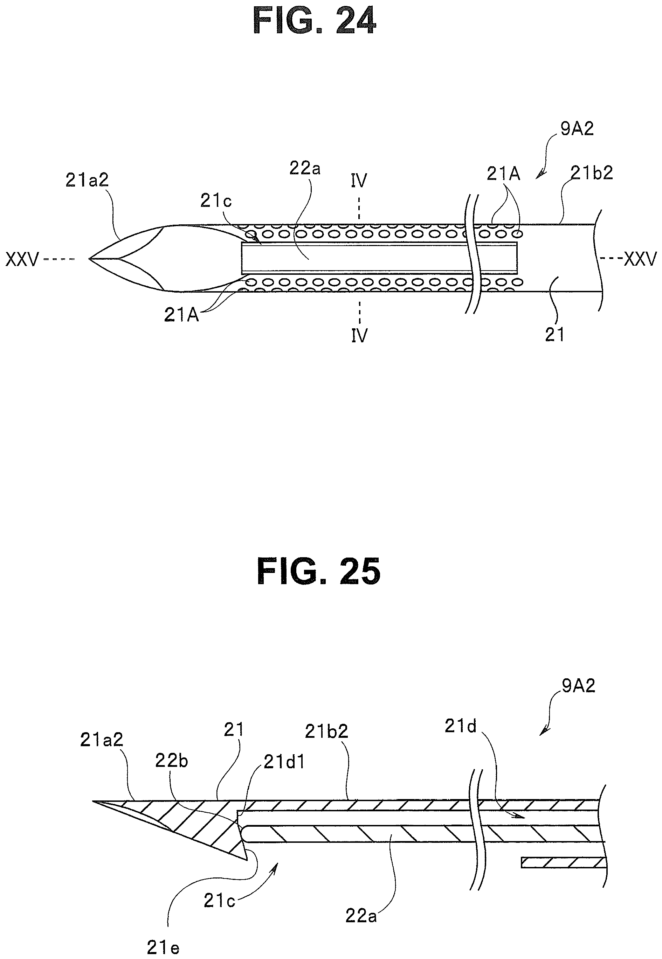

Fig. 24 is a plan view of a needle tube distal end portion 21b2 of a puncture tool insertion portion 9A2 according to the present modification 2.

Fig. 25 is a sectional view taken along line XXV-XXV in Fig. 24. A sectional view of the distal end portion of the puncture tool insertion portion 9A2 taken along line IV-IV of the needle tube distal end portion 21b2 of the present modification 2 is same as Fig. 4.

Even though a distal end portion of the needle tube distal end portion 21 b2 of the present modification 2 is such a bevel-cut shape portion 21a2, effects similar to the effects in the puncture tool in the embodiment explained above are obtained.

### (Modification 3)

Though the blade distal end portion 22a is in a tabular shape in the embodiment and the modifications 1 and 2 explained above, the shape may be a tabular shape having a shaft-shaped expanded portion 22a11 at a distal end portion.

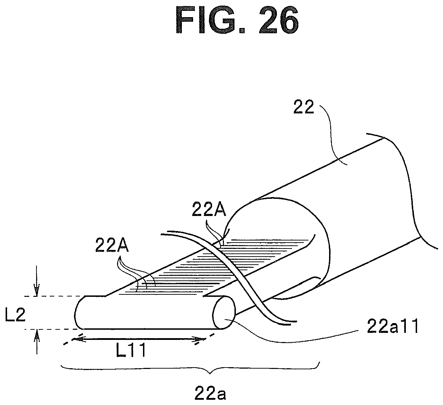

Fig. 26 is a perspective view of the blade distal end portion 22a of the present modification 3. As shown in Fig. 26, the expanded portion 22a11 extends in mutually opposite directions which are orthogonal to an axial direction of the blade distal end portion 22a. The expanded portion 22a11 is formed so that a length L11 of the shaft-shaped expanded portion 22a11 in the direction orthogonal to the axial direction is shorter than an inside diameter of the needle tube 21.

By providing such an expanded portion 22a11, the blade distal end portion 22a does not easily project from the opening portion 21c. That is, the expanded portion 22a11 configures a projection preventing portion which, when the bending portion of the blade distal end portion 22a is formed, restricts the distal end of the blade distal end portion 22a not to move in a side-surface direction of the needle tube 21 after coming into contact with the distal end of the opening portion 21c so that the blade distal end portion 22a does not project from the opening portion 21c.

Even though the blade distal end portion 22a of the present modification 3 has such an expanded portion 22a11 in a shaft shape, effects similar to the effects of the puncture tool in the embodiment explained above are obtained.

### (Modification 4)

Though the blade distal end portion 22a is in a tabular shape in the embodiment and the modifications 1 and 2 explained above, the shape may be a tabular shape having a globe-shaped expanded portion 22a12 at a distal end portion.

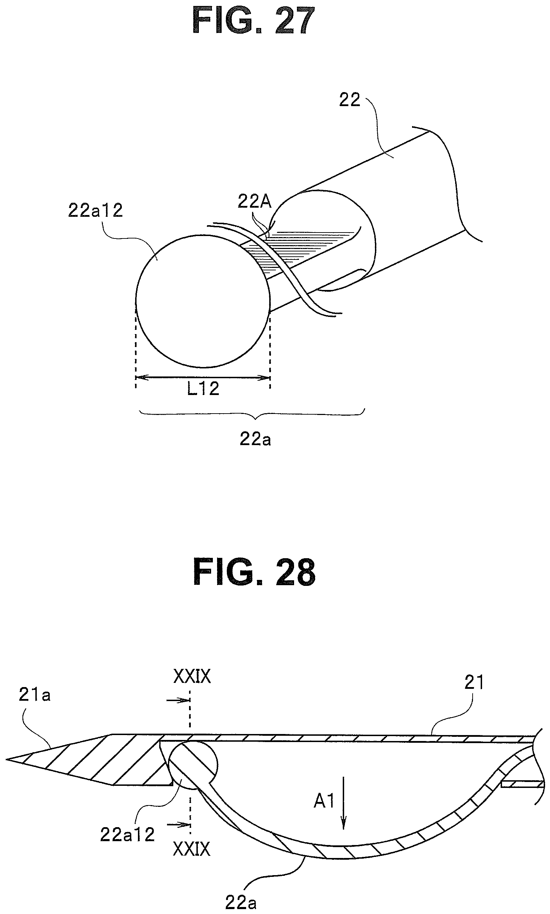

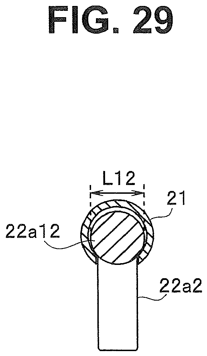

Fig. 27 is a perspective view of the blade distal end portion 22a of the present modification 4. Fig. 28 is a sectional view showing a state in which the globe-shaped expanded portion 22a12 of the blade distal end portion 22a comes into contact with the distal end portion 21d1 of the internal space 21d, and the blade 22 is further pushed out to a distal end side, and the blade distal end portion 22a is bent. Fig. 29 is a sectional view of a distal end portion of the puncture tool insertion portion 9A taken along line XXIX-XXIX in Fig. 28.

As shown in Fig. 27, the globe-shaped expanded portion 22a12 is provided at the distal end of the blade distal end portion 22a. As shown in Fig. 29, the globe-shaped expanded portion 22a12 is formed so that a diameter L12 of the globe-shaped expanded portion 22a12 is shorter than the inside diameter of the needle tube 21. That is, the expanded portion 22a12 configures a projection preventing portion which, when the bending portion of the blade distal end portion 22a is formed, restricts the distal end of the blade distal end portion 22a not to move in a side-surface direction of the needle tube 21 after coming into contact with the distal end of the opening portion 21c so that the blade distal end portion 22a does not project from the opening portion 21c.

By providing such an expanded portion 22a12, the blade distal end portion 22a does not easily project from the opening portion 21c. Even though the blade distal end portion 22a2 of the present modification 4 has such a globe-shaped expanded portion 22a12, effects similar to the effects of the puncture tool in the embodiment explained above are obtained.

### (Modification 5)

Though the high frequency power supply device has two connectors, the connector 8a for a high frequency current to be caused to flow through the blade 22 and the connector 8b for a high frequency current to be caused to flow through the needle tube 21, in the embodiment and the modifications 1 to 4 explained above, the high frequency power supply device may be adapted to have one connector.

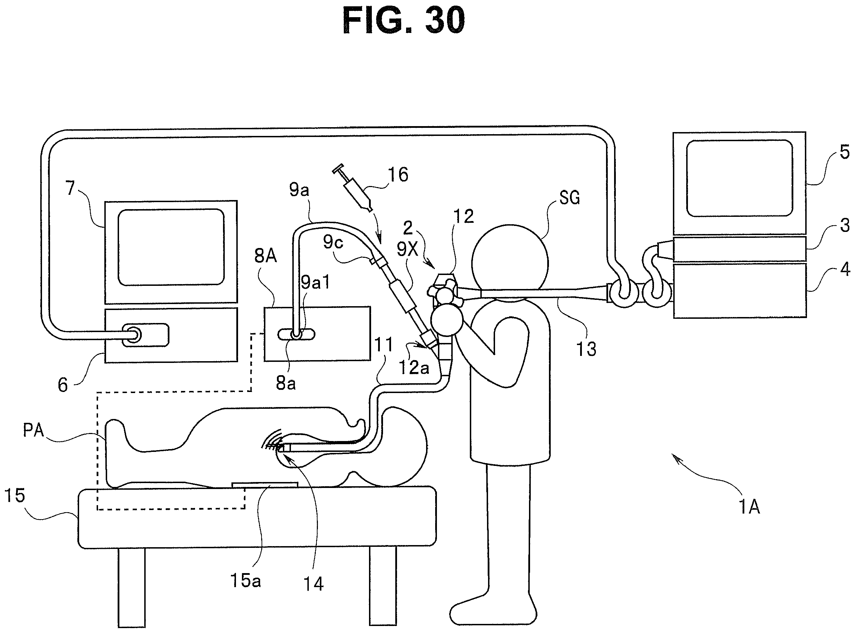

Fig. 30 is a configuration diagram showing a configuration of an operation system 1A using an ultrasound endoscope according to the present modification 5. A high frequency power supply device 8A is provided with only the connector 8a for connecting to a puncture tool 9X. A puncture tool operation portion 9BX of the puncture tool 9X is provided with a switch 9c which is a switch lever for supplying a high frequency current to either the needle tube 21 or the blade 22. Therefore, the surgeon can supply a high frequency current from the high frequency power supply device 8A to the needle tube 21 or the blade 22 by operating the switch 9c to switch. That is, the switch 9c configures a switching portion which switches between electrical connection between the blade distal end portion 22a which is a cutting portion and the high frequency power supply device 8 which is a power supply, and electrical connection between the needle tube distal end portion 21b having a puncture portion and the high frequency power supply device 8 which is a power supply.

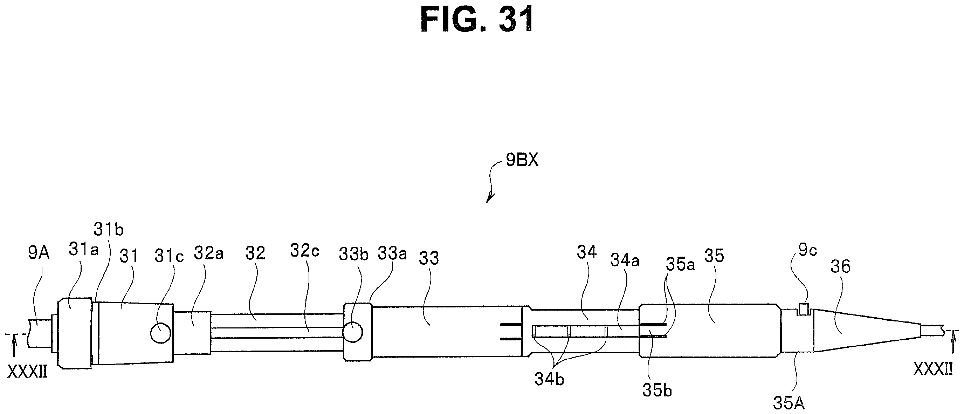

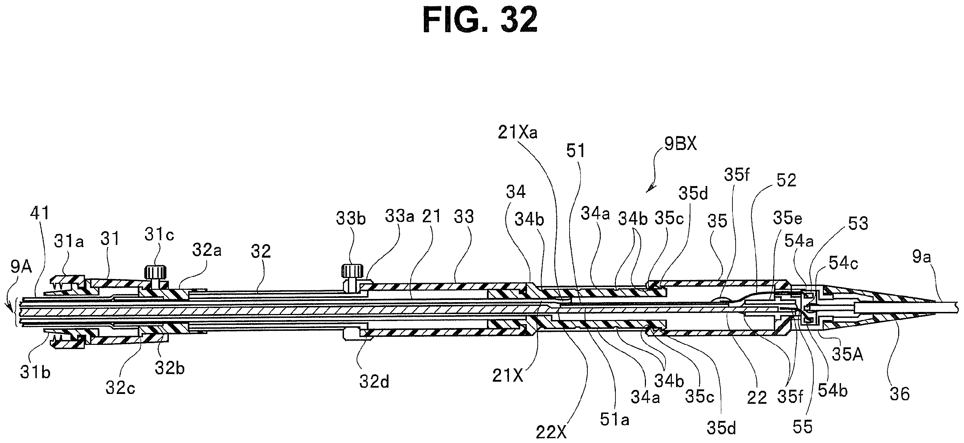

Fig. 31 is an external view of the puncture tool operation portion 9BX according to the present modification 5. Fig. 32 is a sectional view of the puncture tool operation portion 9B taken along line XXXII-XXXII in Fig. 31.

The puncture tool operation portion 9BX has substantially a same configuration as the puncture tool operation portion 9B of the first embodiment explained above. Same components are denoted by same reference signs, and explanation of the components is omitted. In the puncture tool operation portion 9BX, a proximal end side of the blade slider 35 extends, and the switch 9c is provided at an extended portion 35A.

As shown in Fig. 32, a D-cut portion 22X having a D-shaped section is formed at a proximal end portion of the blade distal end portion 22a. A needle-tube extended portion 21X which is extended on a proximal end side is provided at a proximal end portion of the needle tube 21, and a bent contact point portion 21Xa is formed on a proximal end of the needle-tube extended portion 21X.

The D-cut portion 22X of the blade 22 is provided with an insulator 51 a provided with an elongated electrode portion 51 on its surface, at a position where the contact point portion 21Xa is in contact with the D-cut portion 22X. The electrode portion 51 is formed along the axial direction of the blade 22. A proximal end side of the electrode portion 51 on a surface of the insulator 51 a is connected to one end of a lead wire 52 by the solder 35f. The other end of the lead wire 52 is connected to one switch terminal 54a of a switch main body 53 provided on the extended portion 35A through holes formed in the blade slider 35 and the extended portion 35A.

A proximal end portion of the blade 22 is connected to one end of a lead wire 55 via the solder 35f in the blade fixing cap 35e. The other end of the lead wire 55 is connected to one switch terminal 54b of the switch main body 53 provided on the extended portion 35A. Further, a signal line of the cable 9a is connected to one switch terminal 54c of the switch main body 53.

When the blade slider 35 is caused to move relative to the needle tube turning operation portion 34, the contact point portion 21Xa of the needle tube 21 slides along the electrode portion 51 while being in contact with the electrode portion 51 formed on the surface of the insulator 51a. In the switch main body 53, switching is performed so that the switch terminal 54a and switch terminal 54c are electrically connected or so that the switch terminal 54b and switch terminal 54c are electrically connected, by operating the switch 9c.

That is, the surgeon can switch between supplying a high frequency current from the high frequency power supply device 8A to the blade 22 or supplying the high frequency current to the needle tube 21, by operating the switch 9c.

Therefore, the surgeon operates the switch 9c so as to supply the high frequency current to the blade 22 at a time of resecting a tumor tissue in the tumor part Pa and operates the switch 9c so as to supply the high frequency current to the needle tube 21 to prevent adhesion of living lesion cells at a time of pulling out the needle tube 21 from the tumor part Pa.

Thus, with the operation system 1 A of the modification 5 also, effects similar to the effects of the puncture tool in the embodiment explained above are obtained.

As explained above, with the puncture tool and the ultrasound endoscope according to the embodiment and the modifications 1 to 5 explained above, it is possible to cause a lesion part to be reduced by reaching an inside of a lesion part and shaving the inside and prevent adhesion of living lesion cells at a time of pulling out from the lesion part.

### (Second Embodiment)

Though the puncture tool of the first embodiment is configured so as to shave an inside of a lesion part by rotating a blade through which a high frequency current is caused to flow, a puncture tool of the present embodiment is configured to shave an inside of a lesion part by an edge portion provided on a blade without causing a high frequency current to flow through the blade.

Configurations of the puncture tool, ultrasound endoscope and operation system of the present embodiment are substantially the same as the configurations of the puncture tool, ultrasound endoscope and operation system of the first embodiment explained above. Same components are denoted by same reference signs, and explanation of the components is omitted. Different components are explained.

The puncture tool of the present embodiment has the needle tube distal end portion 21b having a conical-shaped puncture portion as in Figs. 2 and 3. Further, the puncture tool of the present embodiment is configured such that a blade distal end portion has an edge portion so as to shave an inside of a lesion part by the edge portion when the blade rotates. Therefore, the blade is not energized when the inside of the lesion part is shaved. Therefore, the high frequency power supply device 8 is provided with the connector for causing a high frequency current to flow through a needle tube at a time of pulling out the needle tube but is not provided with the connector for causing a high frequency current to flow through the blade, and the puncture tool is not provided with a cable for causing a high frequency current to flow through the blade.

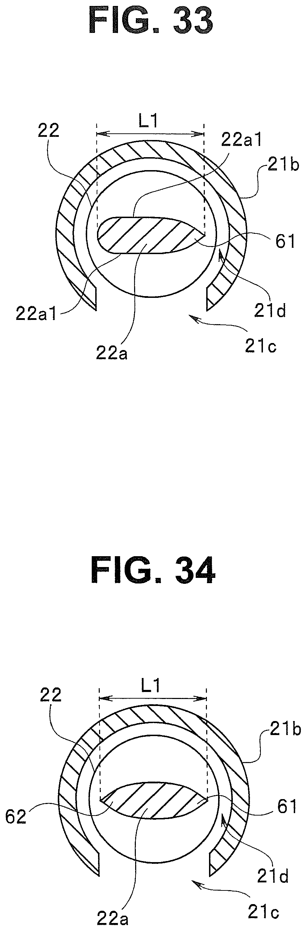

Fig. 33 is a sectional view of a distal end portion of the puncture tool at a time of cutting the needle tube distal end portion 21b with a plane orthogonal to the axial direction of the needle tube 21, according to the present embodiment.

As shown in Fig. 33, an edge portion 61 is formed on the blade distal end portion 22a along the axial direction. Therefore, when the edge portion 61 is applied to a tumor tissue, and the needle tube turning operation portion 34 is caused to rotate so as to shave the tumor tissue, the tumor tissue is resected by the edge portion 61. That is, the edge portion 61 of the bending portion formed when the blade distal end portion 22a projects from the opening portion 21c configures a cutting portion.

Note that the blade distal end portion 22a may be provided with two edge portions. Fig. 34 is a sectional view of a distal end portion of a modification of the puncture tool at a time of cutting the needle tube distal end portion 21b with the plane orthogonal to the axial direction of the needle tube 21 in a case where a blade portion is provided at two positions of the blade distal end portion 22a. An edge portion 62 is formed along a side part opposite to the edge portion 61.

With such a configuration, there is an advantage that the surgeon need not be conscious of a side of the blade distal end portion 22a where an edge portion exists.

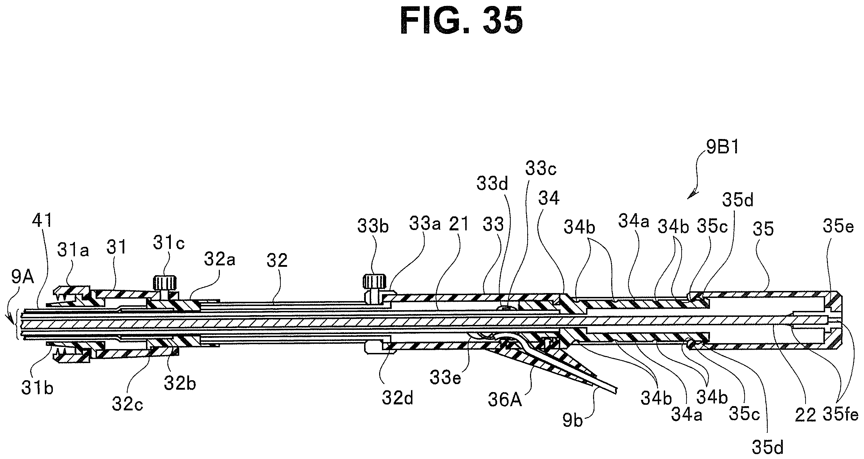

Fig. 35 is a sectional view of a puncture tool operation portion 9B1 along an axial direction according to the present embodiment. In the first embodiment, the blade 22 is connected to be electrically connected to the cable 9a in the puncture tool operation portion 9B. In the present embodiment, however, the proximal end of the blade 22 is not connected with a cable but fixed to the blade fixing cap 35e by adhesive 35fe. Note that, in a case of the present embodiment, the high frequency power supply device may be such that has only one connector as shown in Fig. 30.

Treatment by the puncture tool and ultrasound endoscope of the present embodiment is similar to that in the first embodiment except a point that a tumor tissue is resected without energizing the blade 22. That is, the needle tube 21 is punctured into the tumor part Pa under the ultrasound guide (S1); a tumor tissue is resected by the edge portion 61 (or 62) explained above (S2); the blade 22 is pulled out from the needle tube, and saline or ethanol is injected into the tumor part Pa under the ultrasound guide (S3); and the resected tumor tissue is collected together with the saline or ethanol under the ultrasound guide (S4). Then, the needle tube 21 is pulled out from the tumor tissue while a high frequency current is being given to the needle tube 21 (S5).

Note that, when the manipulation of resecting the tumor tissue is performed while ethanol is being injected, at S2, a stanching effect can be obtained.

Further, note that the tumor tissue of the tumor part Pa may be resected by the edge portion 61 (or 62) by connecting the proximal end of the blade 22 to an ultrasound transducer to cause the blade 22 to vibrate by ultrasound vibration. In this case, the ultrasound vibration may be given while ethanol is being injected into the tumor part Pa.

Therefore, it is possible to reduce the tumor part Pa by shaving the tumor tissue in the tumor part Pa with the edge portion 61 (or 62) and prevent adhesion of living lesion cells at a time of pulling out the needle tube 21 from a lesion part while causing a high frequency current to flow through the needle tube 21.

Note that, as for the present embodiment also, the configuration of each of the modifications 1 to 4 of the first embodiment is applicable.

### (Third Embodiment)

A puncture tool of the present third embodiment is also configured so as to shave an inside of a lesion part by an edge portion provided on a blade without causing a high frequency current to flow through the blade like the blade of the second embodiment. The puncture tool of the present third embodiment is, however, configured such that a blade distal end portion is not bent from an opening portion of a needle tube distal end portion but the blade projects in an oblique direction relative to an axial direction of the needle tube distal end portion so that an inside of a lesion part is shaved with an edge portion provided at a distal end of the blade.

Configurations of the puncture tool, ultrasound endoscope and operation system of the present embodiment are substantially the same as the configurations of the puncture tool, ultrasound endoscope and operation system of the second embodiment explained above. Same components are denoted by same reference signs, and explanation of the components is omitted. Different components are explained.

Further, the puncture tool of the present embodiment is configured such that the blade distal end portion has the edge portion so as to shave an inside of a lesion part by the edge portion when the blade rotates. Therefore, the blade is not energized when the inside of the lesion part is shaved. Therefore, the high frequency power supply device 8 is provided with the connector for causing a high frequency current to flow through a needle tube at a time of pulling out the needle tube but is not provided with the connector for causing a high frequency current to flow through the blade, and the puncture tool is not provided with a cable for causing a high frequency current to flow through the blade.

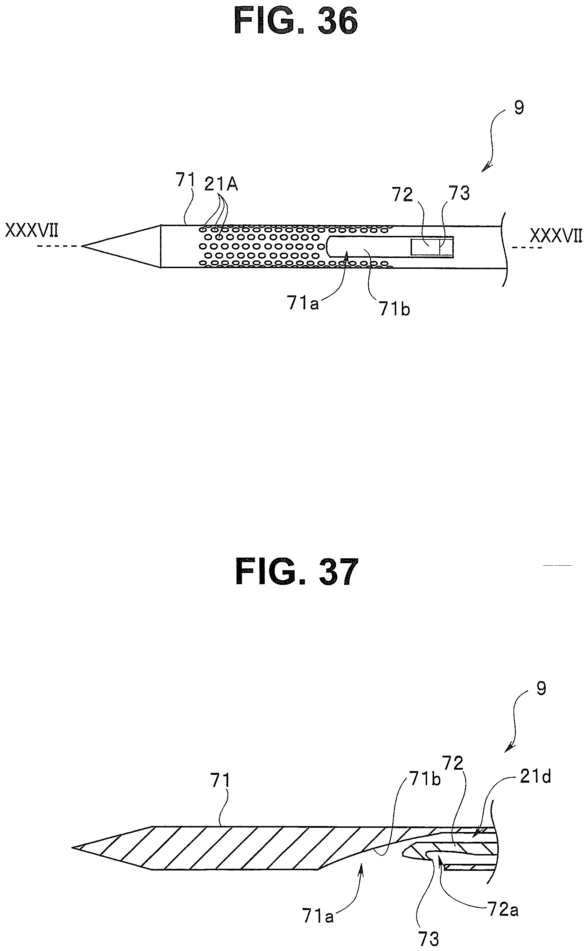

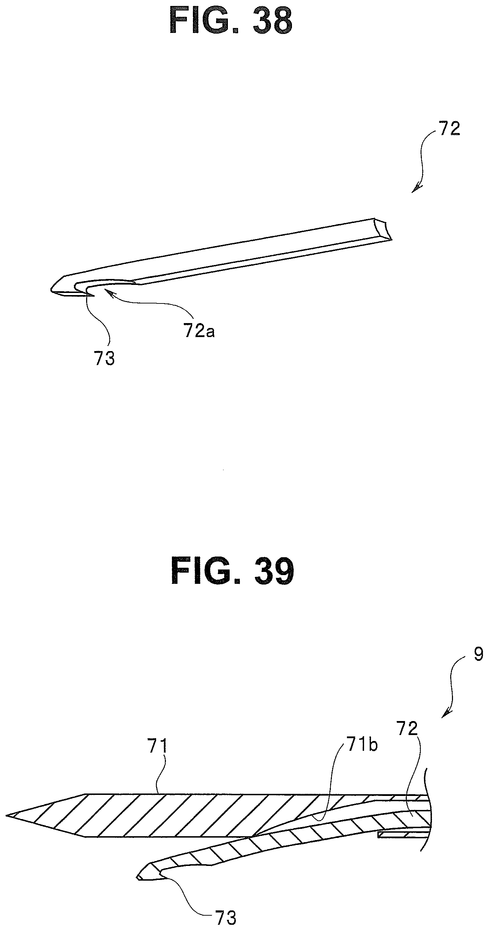

Fig. 36 is a plan view of a needle tube distal end portion 71 according to the present embodiment. Fig. 37 is a sectional view of the needle tube distal end portion 71 taken along line XXXVII-XXXVII in Fig. 36. Fig. 38 is a perspective view of the blade distal end portion according to the present embodiment. Fig. 39 is a sectional view of the needle tube distal end portion 71 at a time when the blade distal end portion projects from an opening portion.

The needle tube distal end portion 71 of the needle tube 21 has a conical-shaped distal end portion.

An inclined portion 71d having a gently inclined surface toward an opening portion 71 a is provided on a distal end side of the internal space 21 d of the needle tube distal end portion 71.

As shown in Figs. 37 and 38, a concave portion 72a is formed on a distal end side of a blade distal end portion 72 of the blade 22 inserted into the internal space 21d of the needle tube 21, and an edge portion 73 is formed on a distal end side of the concave portion 72a. As shown in Fig. 37, the blade distal end portion 72 is in a hook shape, and an edge of the edge portion 73 existing at a distal end of the hook shape is formed such that it faces to a proximal end side. That is, the edge portion 73 configures a cutting portion.

In the blade distal end portion 72, ultrasound reflection processing is applied only to a part around the concave portion 72a though it is not shown. In the needle tube distal end portion 71, dimple processing with a plurality of dimples 21A is applied to a surface of the needle tube distal end portion 71 as an ultrasound reflection processing portion along an axial direction of the needle tube distal end portion 71 within a range where the blade distal end portion 72 can project from the opening portion 71a to resect a tumor tissue, as explained later.

With the puncture tool 9 having the configuration as explained above, it is possible to resect a tumor tissue in the tumor part Pa in such a manner that the tumor tissue is shaved with the edge portion 73, by causing the blade distal end portion 72 to project from the opening portion 71 a and moving the blade 22 or the needle tube 21 so as to cause the blade distal end portion 72 to move backward or forward along the axial direction.

Treatment by the puncture tool and ultrasound endoscope of the present embodiment is similar to that in the first embodiment except the point that a tumor tissue is resected without energizing the blade 22. That is, the needle tube distal end portion 71 is punctured into the tumor part Pa under the ultrasound guide (S1); a tumor tissue is resected by the edge portion 73 explained above (S2); the blade distal end portion 72 is pulled out from the needle tube 21, and saline or ethanol is injected into the tumor part Pa under the ultrasound guide (S3); and the resected tumor tissue is collected together with the saline or ethanol under the ultrasound guide (S4). Then, the needle tube 21 is pulled out from the tumor tissue while a high frequency current is being given to the needle tube 21 (S5).

Note that, when the manipulation of resecting the tumor tissue is performed while ethanol is being injected, at S2, the stanching effect can be obtained.

Therefore, it is possible to reduce the tumor part Pa by shaving the tumor tissue in the tumor part Pa with the edge portion 73 and prevent adhesion of living lesion cells at a time of pulling out the needle tube 21 from a lesion part while causing a high frequency current to flow through the needle tube 21.

### (Modifications)

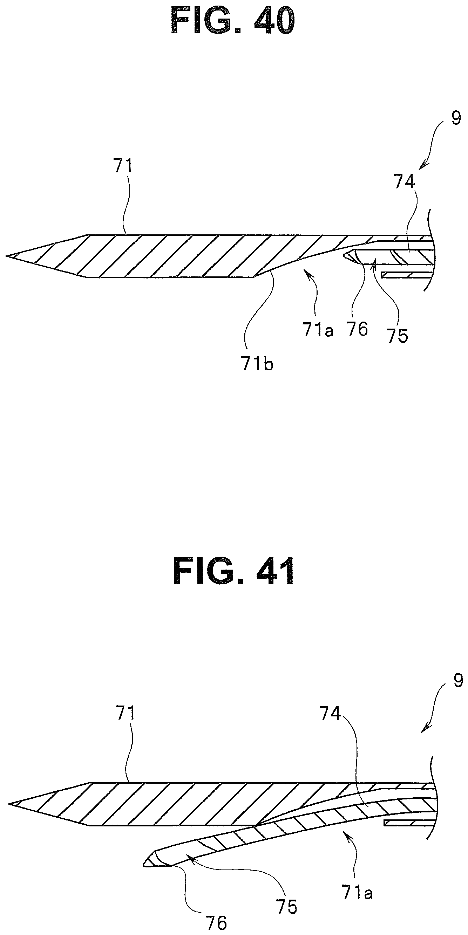

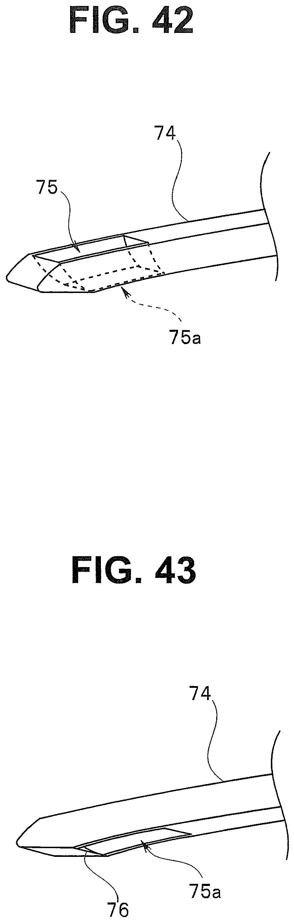

Fig. 40 is a sectional view along an axial direction of a needle tube distal end portion according to a modification of the present third embodiment. Fig. 41 is a sectional view of the needle tube distal end portion along the axial direction of a needle tube at a time when the blade distal end portion in Fig. 40 projects from the opening portion. Fig. 42 is a perspective view of a blade distal end portion 74 of the blade 22 seen from an upper surface side. Fig. 43 is a perspective view of the blade distal end portion 74 seen from a lower surface side.

As shown in Figs. 40 to 43, a hole 75 is formed on a distal end side of the blade distal end portion 74 of the present modification, and an edge portion 76 is provided on one opening portion 75a on a distal end side of the hole 75. The edge portion 76 is formed toward a proximal end side. That is, the edge portion 76 configures a cutting portion.

In the present modification also, ultrasound reflection processing is applied only to a part around the opening portion 75a where the edge portion 76 exists, on the blade distal end portion 74.

With the puncture tool 9 of the present modification, it is possible to resect a tumor tissue in the tumor part Pa in such a manner that the tumor tissue is shaved with the edge portion 76, by causing the blade distal end portion 74 to project from the opening portion 71 a and moving the blade 22 or the needle tube 21 so as to cause the blade distal end portion 74 to advance or retreat along the axial direction.

Note that, as for the present embodiment also, the configuration of each of the modifications 1 to 4 of the first embodiment is applicable.

As explained above, with the puncture tool and ultrasound endoscope of the first to third embodiments and each modification explained above, it is possible to cause a lesion part to be reduced by the puncture tool being punctured into a lesion part and shaving an inside of the lesion part and prevent adhesion of living lesion cells at a time of pulling out from the lesion part.

Further, since it is possible to reduce a size of a tumor and prevent adhesion of living lesion cells, it is possible to perform an operation for excising a tumor part immediately after that.

Note that, though an example of reduction of the tumor part Pa of the pancreas P has been explained in the first to third embodiments and each modification explained above, the puncture tools explained above can be used for reduction of a lesion part of other organs, for example, a liver.

Further, note that, though causing a high frequency current to flow through a needle tube is performed to prevent adhesion of living lesion cells at a time of pulling out the needle tube 21 from a lesion part in the first to third embodiments and each modification explained above, causing a high frequency current to flow may be performed at a time of puncturing the needle tube 21 if a surface of the lesion part is hard.

The present invention is not limited to the embodiments explained above. Various modifications, improvements and the like are possible within a range not departing from the spirit of the present invention.

This application is made, claiming priority based on Japanese Patent Application No. 2012-271651 applied to Japan on December 12, 2012, the disclosed contents of which are incorporated in the specification and claims of this application.

## Claims

1. A puncture tool (9) comprising:
a tubular portion (21) internally including a channel whose distal end side opens on a side surface;
a suction device connecting portion arranged on a proximal end side of the tubular portion so as to communicate with the channel;
a puncture portion (21a) to be punctured into a subject, the puncture portion being arranged on a distal end face of the tubular portion;
an opening portion of the channel provided on a side surface of the puncture portion;
a blade (22) capable of being inserted through the channel, and forming a cutting portion to be exposed from the opening in a state of being bent by a distalmost end part of a distal end portion coming into contact with a distal end side inner wall portion of the channel when being inserted through the channel; and
a first power supply (8) connecting portion for electrically connecting the puncture
portion and a power supply.

2. The puncture tool according to claim 1, comprising a second power supply connecting portion for electrically connecting the cutting portion of the blade and the power supply.

3. The puncture tool according to claim 2 comprising a switching portion switching between electrical connection between the cutting portion of the blade and the power supply and electrical connection between the puncture portion and the power supply.

4. The puncture tool according to claim 2, wherein electrical resistance of the tubular portion is higher than electrical resistance of the blade.

5. The puncture tool according to claim 4, wherein
the tubular portion is made of metal; and
an oxidized layer obtained by oxidizing the metal is arranged on an inner periphery of the channel.

6. The puncture tool according to claim 1, wherein the blade includes a projection preventing portion restricting, when the cutting section in a bent shape is formed, movement of the distalmost end part in a side-surface direction of the tubular portion after coming into contact with the distal end side inner wall portion of the channel so that a distal end of the blade does not project from the opening portion.

7. The puncture tool according to claim 1, wherein an end surface on a puncture portion side of the opening portion has such an inclined surface that a section on the puncture portion side of the opening portion forms an acute angle when the cutting section in the bent shape is formed to prevent the distalmost end part from projecting from the opening portion.

8. The puncture tool according to claim 1, wherein ultrasound reflection processing is applied to a surface of the puncture portion around the opening portion and a surface of the cutting portion.

9. The puncture tool according to claim 1, comprising a suction device connecting portion arranged on a proximal end side of the tubular portion so as to communicate with the channel and connected to a suction device.

10. An ultrasound endoscope comprising:
the puncture tool according to claim 1; and
an ultrasound observation portion transmitting ultrasound toward the puncture tool and receiving the ultrasound reflected from the puncture tool.

## Patentansprüche

1. Punktionsinstrument (9) mit
einem rohrförmigen Abschnitt (21), der in seinem Inneren einen Kanal aufweist, dessen distale Endseite an einer Seitenfläche offen ist;
einem Saugvorrichtungverbindungsabschnitt, der an einer proximalen Endseite des rohrförmigen Abschnitts so angeordnet ist, dass er mit dem Kanal in Verbindung steht;
einem Punktionsbereich (21a) zum Punktieren eines Patienten, wobei der Punktionsbereich an einer distalen Endfläche des rohrförmigen Abschnitts angeordnet ist;
einem Öffnungsbereich des Kanals, der an einer Seitenfläche des Punktionsbereichs vorgesehen ist;
einem Messer (22), das dazu ausgebildet ist, durch den Kanal eingeführt zu werden und das in einem Zustand des Gebogen-Seins durch ein dem distalen Ende am nächsten liegendes Endteil eines distalen Endbereichs, der beim Einführen durch den Kanal mit einem an der distalen Endseite gelegenen Innnenwandbereich des Kanals in Kontakt kommt, einen aus der Öffnung freizulegenden Schneidbereich bildet; und
einem ersten Verbindungsbereich für eine Energiezufuhr (8), um den Punktionsbereich elektrisch mit der Energiezufuhr zu verbinden.

2. Punktionsinstrument nach Anspruch 1, mit einem zweiten Verbindungsbereich für die Energiezufuhr, um den Schneidbereich des Messers elektrisch mit der Energiezufuhr zu verbinden.

3. Punktionsinstrument nach Anspruch 2, mit einem Schaltbereich zum Schalten zwischen einer elektrischen Verbindung des Schneidbereichs des Messers mit der Energiezufuhr und einer elektrischen Verbindung des Punktionsbereichs mit der Energiezufuhr.

4. Punktionsinstrument nach Anspruch 2, bei dem der elektrische Widerstand des rohrförmigen Abschnitts größer ist als der elektrische Widerstand des Messers.

5. Punktionsinstrument nach Anspruch 4, wobei
der rohrförmigen Abschnitt aus Metall besteht; und
eine durch Oxidieren des Metalls erhaltene oxidierte Schicht an einem Innenumfang des Kanals angeordnet ist.

6. Punktionsinstrument nach Anspruch 1, wobei das Messer einen Überstandverhinderungsbereich aufweist, der, beim Ausformen des Schneidbereichs in eine gebogene Kontur, eine Bewegung des dem distalen Ende am nächsten liegenden Endteils in Richtung einer Seitenfläche des rohrförmigen Abschnitts beschränkt, nach Inkontaktkommen mit dem an der distalen Endseite gelegenen Innnenwandbereich des Kanals, so dass ein distales Ende des Messers nicht über den Öffnungsbereich vorsteht.

7. Punktionsinstrument nach Anspruch 1, wobei eine Endfläche an der Punktionsbereichseite des Öffnungsbereichs eine Fläche aufweist, die derart geneigt ist, dass ein Abschnitt auf der Punktionsbereichseite des Öffnungsbereichs einen spitzen Winkel bildet, wenn der Schneidbereich in die gebogene Kontur ausgeformt wird, um das dem distalen Ende am nächsten liegenden Endteil daran zu hindern, über den Öffnungsbereich vorzustehen.

8. Punktionsinstrument nach Anspruch 1, wobei eine rund um den Öffnungsbereich gelegene Fläche des Punktionsbereichs und eine Fläche des Schneidbereichs einer Ultraschallreflexionsbearbeitung unterzogen werden.

9. Punktionsinstrument nach Anspruch 1, mit einem Saugvorrichtungverbindungsabschnitt, der an einer proximalen Endseite des rohrförmigen Abschnitts angeordnet ist, um eine Verbindung mit dem Kanal herzustellen, und der mit einer Saugvorrichtung verbunden ist.

10. Ultraschallendoskop mit:
einem Punktionsinstrument nach Anspruch 1; und
einem Ultraschallbeobachtungsbereich, der Ultraschall zu dem Punktionsinstrument hin überträgt und den von dem Punktionsinstrument reflektierten Ultraschall empfängt.

## Revendications

1. Outil de ponction (9) comprenant :
une partie tubulaire (21) comprenant de manière interne un canal dont un côté d'extrémité distale s'ouvre sur une surface latérale ;
une partie de connexion de dispositif d'aspiration agencée sur un côté d'extrémité proximale de la partie tubulaire de façon à communiquer avec le canal ;
une partie de ponction (21a) destinée à perforer un sujet, la partie de ponction étant agencée sur une face d'extrémité distale de la partie tubulaire ;
une partie d'ouverture du canal prévue sur une surface latérale de la partie de ponction ;
une lame (22) capable d'être insérée à travers le canal, et formant une partie de découpe destinée à être exposée depuis l'ouverture dans un état de courbure par une partie d'extrémité la plus distale de la partie d'extrémité distale venant en contact avec une partie de paroi interne du côté d'extrémité distale du canal lors de l'insertion à travers le canal ; et
une première partie de connexion d'alimentation (8) destinée à connecter électriquement la partie de ponction et une alimentation.

2. Outil de ponction selon la revendication 1, comprenant une deuxième partie de connexion d'alimentation destinée à connecter électriquement la partie de découpe de la lame et l'alimentation.

3. Outil de ponction selon la revendication 2, comprenant une partie de commutation commutant entre une connexion électrique entre la partie de découpe de la lame et l'alimentation et une connexion électrique entre la partie de ponction et l'alimentation.

4. Outil de ponction selon la revendication 2, dans lequel la résistance électrique de la partie tubulaire est supérieure à la résistance électrique de la lame.

5. Outil de ponction selon la revendication 4, dans lequel
la partie tubulaire est composée de métal ; et
une couche oxydée obtenue en oxydant le métal est agencée sur une périphérie interne du canal.

6. Outil de ponction selon la revendication 1, dans lequel la lame comprend une partie de prévention de projection limitant, lorsque la section de découpe est formée dans une forme courbée, le déplacement de la partie d'extrémité la plus distale dans une direction de surface latérale de la partie tubulaire après être venue en contact avec la partie de paroi interne de côté d'extrémité distale du canal de sorte qu'une extrémité distale de la lame ne fait pas saillie depuis la partie d'ouverture.

7. Outil de ponction selon la revendication 1, dans lequel une surface d'extrémité sur un côté de partie de ponction de la partie d'ouverture présente une surface inclinée d'une manière telle qu'une section sur le côté de la partie de ponction de la partie d'ouverture forme un angle aigu lorsque la section de découpe est formée dans la forme courbée pour empêcher la partie d'extrémité la plus distale de faire saillie depuis la partie d'ouverture.

8. Outil de ponction selon la revendication 1, dans lequel un traitement de réflexion des ultrasons est appliqué sur une surface de la partie de ponction autour de la partie d'ouverture et une surface de la partie de découpe.

9. Outil de ponction selon la revendication 1, comprenant une partie de connexion de dispositif d'aspiration agencée sur un côté d'extrémité proximale de la partie tubulaire de façon à communiquer avec le canal et connectée à un dispositif d'aspiration.

10. Endoscope ultrasonore comprenant :
l'outil de ponction selon la revendication 1 ; et
une partie d'observation par ultrasons transmettant des ultrasons vers l'outil de ponction et recevant les ultrasons réfléchis depuis l'outil de ponction.
